# EUROPEAN PATENT APPLICATION

(11) **EP 3 348 254 A1**
(43) Date of publication of application: **18.07.2018**
(21) Application number: 16844684.7
(22) Date of filing: 07.09.2016
(51) Int. Cl.: A61K 8/97, A61K 36/752, A61K 36/12, A61K 36/185, A23L 33/105, A61Q 19/00

(54) **COMPOSITION FOR ALLEVIATING SKIN INFLAMMATION CAUSED BY YELLOW DUST AND FINE PARTICULATE, COMPRISING NATURAL PLANT EXTRACT**

(30) Priority: 08.09.2015 KR 20150126832
(71) Applicant: Celltrion Inc., Incheon 22014 (KR); Durae Corporation, Gunpo-si, Gyeonggi-do 15847 (KR); University - Industry Cooperation Group of Kyung Hee University, Yongin-si, Gyeonggi-do 17104 (KR)
(72) Inventor: KIM, Jae Hun, Goyang-si Gyeonggi-do 10407 (KR); LEE, Seung Ki, Incheon 21982 (KR); LIM, Joo Hyuck, Incheon 21986 (KR); KIM, Yeon Sook, Bucheon-si Gyeonggi-do 14508 (KR); MOON, Won Kang, Seoul 07993 (KR); CHOI, Chi Ho, Incheon 22201 (KR); YOON, Kwang Jun, Incheon 22760 (KR); MOON, Sung Ho, Yongin-si Gyeonggi-do 16892 (KR); LEE, Sung Hyun, Gunpo-si Gyeonggi-do 15815 (KR); YEOM, Myeong Hun, Yongin-si Gyeonggi-do 16822 (KR); AHN, Soo Mi, Suwon-si Gyeonggi-do 16708 (KR); KIM, Kyoung Wook, Jeju-si Jeju-do 63099 (KR); SUNG, Mi Kyung, Incheon 21319 (KR); JANG, Young Pyo, Seoul 06063 (KR); KIM, Jinju, Gwacheon-si Gyeonggi-do 13806 (KR)
(74) Representative: AWA Sweden AB
(86) International application number: PCT/KR2016/010037
(87) International publication number: WO 2017/043868

(57) **Abstract**

The present invention relates to a composition comprising natural plant extracts for alleviating inflammation caused by yellow dust and fine particulate, wherein the plant extracts of the present invention inhibit a production of NO caused by the yellow dust and fine particulate and a production of inflammatory cytokines, thus having an excellent effect on preventing or alleviating skin inflammation caused by the yellow dust, fine particulate and other harmful substances.

## Description

### Technical Field

The present invention relates to a composition comprising natural plant extract for alleviating skin inflammation caused by yellow dust and fine particulate, and more particularly, to a composition for alleviating skin inflammation caused from harmful atmospheric environments, comprising one or more extracts selected from the group consisting of a *Citrus sunki* peel extract, a *Sceptridium ternatum* (or *Botrychium ternatum*) extract and a *Korthalsellajaponica* extract as an effective ingredient.

### Background

Inflammatory responses in skin begin to take place as an action for defending the skin from damages, when they are caused by physical stimuli, chemical substances, germs, etc. In response, keratinocytes or Langerhans cells send out various types of cytokine, thereby launching such inflammatory responses. Interleukin-8 (IL-8), interleukin-6 (IL-6), interleukin-1 (IL-1), tumor necrosis factor-a (TNF-α), etc. are representative of the cytokine, by which activated macrophages excessively produce nitricoxide (NO) or prostaglandin E2 (PGE2) to aggravate an inflammatory process more.

Fine particulate collectively refers to dusts having a particle size of 10 *µ*m or less and its abbreviation is PM10 (Particulate Matter 10), which also includes PM2.5 (Particulate Matter 2.5), ultra-fine particulate having a particle size of 2.5 *µ*m or less. The fine particulate mainly occurs when burning fossil fuel, and a considerable portion thereof is occupied by the one coming from China, of which dependence on coal accounts for 70%. Moreover, yellow dust refers to a phenomenon in which fine sand, yellow soil or dust in deserts and yellow soil zones at the center of Asia Continent including China, Mongol or the like float in the sky, fly away by the upper wind and drop in remote areas. These long-range migratory air pollutants can move far away down to Jeju Island.

It is estimated that about 25 to 33% of diseases in industrial countries are caused by environmentally hazardous factors and that about 310,000 people per year die early due to polluted air in Europe. The American Cancer Society announced its research results that a rise in ultra-fine particulate by 10 *µ*g per m3 results in 7% increase in a total death rate. According to results of a joint research (Journal of Allergy and Clinical Immunology. 132(2), 2013, 495-498) conducted in 2013 by the Ministry of Environment, it was shown that a rising concentration of the fine particulate or the air pollutants aggravates dermatitis symptoms more. Whenever the fine particulate (PM10) was increased by 1 *µ*g/m³, the dermatitis symptoms rose by 0.4% on average, too. As total volatile organic compounds (TVOC) and benzene, ones of air pollutants, were increased by 0.1 ppb, the symptoms rose 2.59% and 2.74% on average, respectively. Also, if the yellow dust occurs, its cloud of dust contaminates air, which turns yellowish brown, and an amount of dust in air increases four times on average, thus making skin easily tired and lifeless. What is worse, the yellow dust has a smaller particle size than other general dusts, such that it can penetrate deep into pores of the skin, thus causing skin troubles and more aggravating various symptoms such as dry skin, irritant dermatitis, allergic dermatitis, acne, freckles, melasma, etc. Thus, it is thought that there will be a rise in an occurrence of dermatitis caused by the yellow dust and the fine particulate and there will be a growing need for a material that can alleviate skin inflammatory responses caused by the yellow dust and the fine particulate.

Steroids are mainly used to treat skin inflammation, but their long-term administration causes various side effects such as skin atrophy, potential growth retardation or the like. Thus, non-steroids have recently been used more. However, the non-steroids exhibit side effects such as symptoms like erythema, itchiness, etc., weak immunity and the like, thus it is still difficult to fundamentally treat dermatitis. Thus, there have been a number of researches conducted to find novel substances with a high remedial effect and less side effects from natural product-derived materials, which have been proven safe. Representatively there are *Sorbus commixta* extract (KR 10-0563548), *Gallnut* extract (KR 10-1309172), *Spirulina* extract (KR 10-2009-0079468), *Glehnia littoralis* extract (KR 10-0919852), etc., but there has not been developed yet an extract having an effect of alleviating inflammation caused by the yellow dust and the fine particulate.

Accordingly, there has been a growing need for a novel substance with a high remedial effect and less side effects from natural plant-derived materials, which have been proven safe, but there have not been any reported study results about the natural plant-derived materials having an effect of alleviating inflammation caused by the yellow dust and the fine particulate.

### Detailed Description of the Invention

### Technical Problem

Accordingly, as a result of conducting an exemplary research on natural product-derived materials capable of alleviating skin inflammation caused by yellow dust and fine particulate and solving problems of existing skin anti-inflammatory agents, the present inventors have identified that a *Citrus sunki* peel extract, a *Sceptridium ternatum* (or *Botrychium ternatum*) extract and a *Korthalsella japonica* extract alleviate skin inflammation caused by the yellow dust and the fine particulate, thus completing the present invention.

Thus, an object to be solved by the present invention is to provide a composition for alleviating skin inflammation caused from the yellow dust or the fine particulate, comprising one or more extracts selected from the group consisting of the *Citrus sunki* peel extract, the *Sceptridium ternatum* (or *Botrychium ternatum*) extract and the *Korthalsellajaponica* extract as an effective ingredient.

Also, another object to be solved by the present invention is to provide a method for alleviating skin inflammation by using the above composition.

Further, yet another object to be solved by the present invention is to provide a method for alleviating a degree of skin redness, an amount of skin erythema or an amount of transepidermal water loss, wherein the method comprises a step of applying the above composition to skin.

### Technical Solution

To solve the above objects, the present invention provides a composition for alleviating skin inflammation caused from yellow dust or fine particulate, comprising one or more extracts selected from the group consisting of a *Citrus sunki* peel extract, a *Sceptridium ternatum* (or *Botrychium ternatum*) extract and a *Korthalsella japonica* extract as an effective ingredient.

Also, the present invention provides a method for alleviating skin inflammation by using the above composition.

Further, the present invention provides a method for alleviating a degree of skin redness, an amount of skin erythema or an amount of transepidermal water loss, wherein the method comprises a step of applying the above composition to skin.

Hereinafter, the present invention will be described in more detail.

The present invention provides a composition for alleviating skin inflammation caused from harmful atmospheric environments, comprising one or more extracts selected from the group consisting of a *Citrus sunki* peel extract, a *Sceptridium ternatum* (or *Botrychium ternatum*) extract and a *Korthalsellajaponica* extract as an effective ingredient.

The above *Citrus sunki* peel means a peel of *Citrus sunki,* which is a native mandarin wildly grown in Jeju Island, but it is distinguished from a Japanese species of *Citrus unshiu,* which is popularly used in recent days. Its fruits have such a unique scent and taste that they could belong to a high class of indigenous products of Jeju Island, which were presented to kings in old days. It is called "sanmul" or "sangyul" in Jeju regions and belongs to a Rutaceae family of dicotyledonous plants in the order of Geraniales.

The above *Sceptridium ternatum* (or *Botrychium ternatum)* is also called "flowery gosari (flowery bracken fern)," and it is a perennial fern belonging to an *Ophioglossaceae* family in the order of *Ophioglossales.* It is wildly grown in mountains of Korea, Japan, Taiwan and China and it is 15-40 cm high without fuzz as a whole. Its root is thick and flesh and spreads in all directions, out of which one stem springs and stands upright.

The above *Korthalsella Japonica*, which is an evergreen hemi-parasitic shrub parasitic on camellia, belongs to a *Loranthaceae* family along with *Loranthus chinensis* Danser (mulberry mistletoe), alder tree mistletoe and *Usnea longissima* (pine tree mistletoe). The *Korthalsellajaponica* can be seen on Jeju Island and some islands of the south coast of South Korea. It is 6-15 cm long and its leave has atrophied into a node shape at the both ends of a joint and its branch takes on a green color.

The above skin inflammation may comprise, without limitation, all the skin inflammatory diseases accompanied by inflammatory responses, which may occur to skin in contact with yellow dust or fine particulate, and it may be contact dermatitis, but not limited thereto.

In case of the above skin inflammation, their occurrence may be identified by measuring a degree of skin redness, an amount of skin erythema or an amount of transepidermal water loss, but not limited thereto.

The above extract may be obtained in such a way that it is extracted by means of water, C₁ to C₄ lower carbon alcohols or a mixed solvent thereof, wherein these lower carbon alcohols may be ethanol or methanol, more particularly ethanol.

In one specific embodiment of the present invention, the present invention provides a composition for alleviating skin inflammation caused from harmful atmospheric environments, comprising a *Citrus sunki* peel extract as an effective ingredient.

In one specific embodiment of the present invention, the present invention provides a composition for alleviating skin inflammation caused from harmful atmospheric environments, comprising a *Sceptridium ternatum* (or *Botrychium ternatum*) extract as an effective ingredient.

In one specific embodiment of the present invention, the present invention provides a composition for alleviating skin inflammation caused from harmful atmospheric environments, comprising a *Korthalsellajaponica* extract as an effective ingredient.

In one specific embodiment of the present invention, the present invention provides a composition for alleviating skin inflammation caused from harmful atmospheric environments, further comprising any one or more of the *Citrus sunki* peel extract and the *Sceptridium ternatum* (or *Botrychium ternatum*) extract in addition to the *Korthalsellajaponica* extract as an effective ingredient.

In one specific embodiment of the present invention, the present invention provides a composition for alleviating skin inflammation, characterized in that the above extract is i) the *Citrus sunki* peel extract comprising one or more compounds selected from the group consisting of hesperidine, tetramethyl-O-isoscutellarein, nobiletin and tangeretin; ii) the *Sceptridium ternatum* (or *Botrychium ternatum*) extract comprising one or more compounds selected from the group consisting of quercetin-3-O-β-glucosyl(1→2)-α-rhamnoside-7-O-α-rhamnoside, quercetin-3-O-β-glucosyl(1→2)-α-rhamnoside-7-O-β-glucoside, ternatumoside VI, ternatumoside XI and ternatumoside XII; or iii) the *Korthalsellajaponica* extract comprising one or more compounds selected from the group consisting of lucenin-2, vicenin-2 and stellarin-2.

In one specific embodiment of the present invention, the *Citrus sunki* peel extract of the present invention may comprise one or more compounds selected from the group consisting of hesperidine, tetramethyl-O-isoscutellarein, nobiletin and tangeretin as an effective ingredient.

In one specific embodiment of the present invention, the *Sceptridium ternatum* (or *Botrychium ternatum*) extract of the present invention may comprises one or more compounds selected from the group consisting of quercetin-3-O-β-glucosyl(1→2)-α-rhamnoside-7-O-α-rhamnoside, quercetin-3-O-β-glucosyl(1→2)-α-rhamnoside-7-O-β-glucoside, ternatumoside VI, ternatumoside XI and ternatumoside XII as an effective ingredient.

In one specific embodiment of the present invention, the *Korthalsellajaponica* extract of the present invention may comprise one or more compounds selected from the group consisting of lucenin-2, vicenin-2 and stellarin-2 as an effective ingredient.

In one specific embodiment of the present invention, the above extract may be a mixture that combines the *Citrus sunki* peel extract, the *Sceptridium ternatum* (or *Botrychium ternatum*) extract and the *Korthalsellajaponica* extract all together. The ethanol extracts of *Citrus sunki* peel, *Sceptridium ternatum* (or *Botrychium ternatum*) and *Korthalsellajaponica* make a relative difference of effects in inhibiting each production of their inflammation-causing substances. Thus, a use of a mixture that combines all the ethanol extracts of *Citrus sunki* peel, *Sceptridium ternatum* (or *Botrychium ternatum*) and *Korthalsellajaponica* may give a complementary effect on alleviating skin inflammation caused by harmful atmospheric environments mixed with various harmful substances such as yellow dust, fine particulate, etc.

The above inflammation-causing substance means a factor related to inflammatory responses in vivo such as a pro-inflammatory mediator NO or an inflammatory cytokine.

In one specific embodiment of the present invention, in case of the above mixture, its composition ratio of *Citrus sunki* peel extract: *Sceptridium ternatum* (or *Botrychium ternatum*) extract: *Korthalsellajaponica* may be a weight ratio of 1:1:1 to 2:1:1.

In one specific embodiment of the present invention, the above harmful atmospheric environments may be the yellow dust or the fine particulate.

The above harmful atmospheric environments, which comprise air pollutants, refer to atmospheric environments comprising heavy metal, the fine particulate, the yellow dust, etc.

The above yellow dust refers to a phenomenon in which fine sand, yellow soil or dust in deserts and yellow soil zones at the center of Asia Continent including China, Mongol or the like float in the sky, fly away by the upper wind and drop in remote areas as long-range migratory air pollutants, or sandy dust falling down therein.

In one specific embodiment of the present invention, the above fine particulate may comprise one or more fine particulates selected from the group consisting of TSP, PM10 and PM2.5.

In one specific embodiment of the present invention, the above yellow dust or fine particulate may increase a production amount of one or more cytokines selected from the group consisting of G-CSF, GM-CSF, GRO, GRO-α, IL-1α IL-2, IL-3, IL-5, IL-6, IL-8, IL-15, MCP-1, MCP-2 and TNF-α.

The above fine particulate collectively refers to dusts having a particle size of 10 *µ*m or less and its abbreviation is PM10 (Particulate Matter 10), which also includes PM2.5 (Particulate Matter 2.5), ultra-fine particulate having a particle size of 2.5 *µ*m or less. It mainly occurs when burning fossil fuel, and its considerable portion is occupied by fine particulate coming from China, of which dependence on coal accounts for 70%. Also, total suspended particles (TSP) (all dusts generally having a size of 50 *µ*m or less and floating in air) is comprised in the fine particulate in a broad sense.

In one specific embodiment of the present invention, the above extract may inhibit a production of NO, which is a pro-inflammatory mediator.

In one specific embodiment of the present invention, the above extract may reduce a production amount of factors related to inflammatory responses in vivo such as inflammatory cytokines.

In one specific embodiment of the present invention, the above extract may reduce a production amount of IL-1α, IL-2, IL-6, IL-8, GRO-α, GM-CSF and TNF-α as representative inflammatory cytokines.

The above inflammatory cytokines may be G-CSF (granulocyte colony stimulating factor), GM-CSF (granulocyte-macrophage colony stimulating factor), GRO (growth-regulated protein), GRO-α (growth-regulated protein-α), IL-1α (interleukin-1α), IL-2 (interleukin-2), IL-3 (interleukin-3), IL-5 (interleukin-5), IL-6 (interleukin-6), IL-7 (interleukin-7), IL-8 (interleukin-8), IL-10 (interleukin-10), IL-13 (interleukin-13), IL-15 (interleukin-15), INF-γ (interferon-y), MCP-1 (monocyte chemoattractant protein-1), MCP-2 (monocyte chemoattractant protein-2), MCP-3 (monocyte chemoattractant protein-3), CXCL9 (chemokine ligand 9; MIG), CCL5 (chemokine ligand 5; RANTES), TGF-β1 (transforming growth factor-β1), TNF-α (tumor necrosis factor-a) and TNF-β (tumor necrosis factor-β), but not limited thereto.

In one specific embodiment of the present invention, the above *Citrus sunki* peel extract, the *Sceptridium ternatum* (or *Botrychium ternatum*) extract and the *Korthalsellajaponica* extract may be prepared by means of a manufacturing method comprising following steps, but not limited thereto:
1) adding an extraction solvent to *Citrus sunki* peel, *Sceptridium ternatum* (or *Botrychium ternatum*) or *Korthalsellajaponica* to obtain each extract;
2) filtering each extract of a step 1); and
3) concentrating each filtered extract of a step 2) under reduced pressure and drying each resulting concentrate to prepare powder thereof.

In the above method, the *Citrus sunki* peel, *Sceptridium ternatum* (or *Botrychium ternatum*) or *Korthalsella japonica* of the step 1) may be used a cultivated one, a commercially available one or the like without limitation.

In the above method, an extraction method may be a conventional method in the art, such as filtration, hot water extraction, immersion extraction, reflux extraction, ultrasonic extraction, supercritical fluid extraction, etc., and an extract thereof may be prepared under a conventional condition of temperature and pressure known in the art. Also, the extract may be obtained in such a way that it is extracted by using water, C₁ to C₄ lower carbon alcohols or a mixed solvent thereof as an extraction solvent and the above lower carbon alcohols may be ethanol or methanol. The extract may be obtained by adding the extraction solvent that amounts to 1 to 20 times of each quantity of the *Citrus sunki* peel, *Sceptridium ternatum* (or *Botrychium ternatum*) or *Korthalsella japonica*, particularly 5 to 15 times thereof, but is not limited thereto. Also, an extraction temperature may be 20 to 70°C, particularly 25 to 65°C, and more particularly 30 to 60°C, but is not limited thereto. Furthermore, an extraction time may be 1 to 40 hours, particularly 10 to 30 hours, but is not limited thereto.

In the above method, the extract of the step 2) may be filtered 1 to 5 times. Also, the extraction method may be a conventional method in the art, such as sieve filtration, suction filtration, ultra-filtration, filter press, etc., but is not limited thereto.

In the above method, the concentration under reduced pressure of the step 3) may be performed by means of a vacuum evaporator or a rotary evaporator, but is not limited thereto. Also, the drying may be reduced pressure drying, vacuum drying, boiling drying, spray drying or freeze drying, but is not limited thereto.

In one specific embodiment of the present invention, the above composition may be a cosmetic composition, a pharmaceutical composition or a health functional food composition.

The above cosmetic composition may comprise ingredients conventionally used in a cosmetic composition, wherein as one specific example for these ingredients, this may be conventional adjuvants such as antioxidant, stabilizer, solubilizer, vitamin, pigment and flavoring, as well as carrier.

The above cosmetic composition may be prepared into a formulation conventionally prepared in the art. As one specific example for the cosmetic composition, this may be any one formulation selected from the group consisting of a high-viscosity emulsion formulation, a low-viscosity emulsion formulation and a solubilized formulation, but is not limited thereto. The cosmetic composition may comprise a blend ingredient of the cosmetic composition conventionally used in the art, to which the present invention pertains, depending on a formulation to be prepared. As one specific example, the cosmetic composition may be prepared into a formulation such as solution, suspension, emulsion, paste, gel, cream, lotion, powder, soap, surfactant-containing cleansing oil, powder foundation, emulsion foundation, wax foundation, pack, massage cream, spray, and the like, but is not limited thereto. More particularly, the cosmetic composition may be prepared into a formulation such as skin lotion, nutrition lotion, nutrition cream, massage cream, essence, eye cream, sun lotion, sun cream, makeup base, BB cream, cleansing cream, cleansing foam, cleansing water, pack, a stick-type product, a balm type product, spray or powder. The cosmetic composition may be prepared by further selecting, blending and adding a water phase ingredient, an oil phase ingredient, surfactant, moisturizer, lower carbon alcohols, thickener, a chelating agent, preservative, flavoring, etc., depending on a formulation to be prepared.

The above pharmaceutical composition may further contain pharmaceutical adjuvants such as preservative, stabilizer, hydrating agent or emulsification accelerator, salt and/or buffer for adjusting osmotic pressure, etc., and other therapeutically useful substances, and may be formulated into various forms of oral administration or parenteral administration according to a conventional method. In case of the parenteral administration, this may be a local application to skin, an intravenous infusion, a subcutaneous infusion, an intramuscular infusion, an intraperitoneal infusion, a transdermal administration, etc.

The pharmaceutical composition of the present invention may be a formulation for external use on skin. This formulation for external use on skin may be powder, gel, ointment, cream, lotion, liquid or aerosol formulation, but is not limited thereto.

In case of the above health functional food composition, its formulation is not particularly limited, but this may be formulated into, for example, tablet, granule, drink, caramel, diet bar, etc. The health functional food composition of each formulation may be prepared in such a way that those skilled in the art may select and blend optimal ingredients conventionally used in the art in addition to effective ones without any difficulty according to its formulation or purpose of use. And if such ingredients are applied at the same time with other raw materials, a synergy effect may occur.

In one specific embodiment of the present invention, each of the above *Citrus sunki* peel extract, the *Sceptridium ternatum* (or *Botrychium ternatum*) extract and the *Korthalsellajaponica* extract or mixtures thereof may be comprised 0.001 to 30 wt% with regard to the total weight of the composition. If each of the above *Citrus sunki* peel extract, the *Sceptridium ternatum* (or *Botrychium ternatum*) extract and the *Korthalsellajaponica* extract or mixtures thereof is comprised less than 0.001 wt% with regard to the total weight of the composition, this does not exhibit an effect of alleviating skin inflammation. In case of exceeding 30 wt%, a degree of increase in an effect of alleviating skin inflammation was insignificant with regard to an increase in content, and also it is not economical along with a problem of stability in formulation.

In one specific embodiment of the present invention, it was identified that the *Citrus sunki* peel extract, the *Sceptridium ternatum* (or *Botrychium ternatum*) extract, the *Korthalsellajaponica* extract and mixtures thereof inhibited a production of NO caused by yellow dust or fine particulate, which are harmful atmospheric environments, as well as a production of inflammatory cytokines, thus having an excellent effect on preventing and alleviating skin inflammation caused by the yellow dust, the fine particulate and other pollutants.

In one specific embodiment of the present invention, it was identified through clinical tests that the *Citrus sunki* peel extract, the *Sceptridium ternatum* (or *Botrychium ternatum*) extract, the *Korthalsella japonica* and mixtures thereof show an excellent effect on alleviating skin inflammation caused by fine particulate.

Also, the present invention provides a method for alleviating skin inflammation by using the above composition.

In one specific embodiment of the present invention, the above method may comprise a step of applying the composition to skin.

In one specific embodiment of the present invention, the above skin inflammation may comprise, without limitation, all the skin inflammatory diseases accompanied by inflammatory responses, which may occur to skin in contact with yellow dust or fine particulate, and may be contact dermatitis, but not limited thereto.

Also, the present invention provides a method for alleviating a degree of skin redness, an amount of skin erythema or an amount of transepidermal water loss, comprising a step of applying the above composition to skin.

In one specific embodiment of the present invention, the above method may be characterized in that this may be i) a method for alleviating a degree of skin redness by about 20.0%, ii) a method for alleviating an amount of skin erythema by about 16.8%; or iii) a method for alleviating an amount of transepidermal water loss by about 33.2% in 7 days after an application of a fine particulate solution compared to a control without application.

The term "application" used in the present specifications means all the methods for bringing the inventive composition in contact with an individual skin in any appropriate means, through which the present invention aims to let the composition absorbed into the skin.

### Advantageous Effects

The natural plant extracts of the present invention inhibit a production of NO caused by yellow dust and fine particulate and a production of inflammatory cytokines, thus having an excellent effect on preventing or alleviating skin inflammation caused by the yellow dust, the fine particulate and other harmful substances.

### Brief Description of the Drawings

FIGS. 1 to 3 are graphs indicating effects of ethanol extracts of *Citrus sunki* peel (FIG. 1), *Sceptridium ternatum* (or *Botrychium ternatum*) (FIG. 2) and *Korthalsella japonica* (FIG. 3) on inhibiting productions of NOs caused by yellow dust, TSP, PM10 and PM2.5.
FIGS. 4 to 6 are graphs indicating effects of ethanol extracts of *Citrus sunki* peel (FIG. 4), *Sceptridium ternatum* (or *Botrychium ternatum*) (FIG. 5) and *Korthalsella japonica* (FIG. 6) on inhibiting productions of IL-8 caused by yellow dust and PM2.5.
FIGS. 7 to 9 are graphs indicating effects of ethanol extracts of *Citrus sunki* peel (FIG. 7), *Sceptridium ternatum* (or *Botrychium ternatum*) (FIG. 8) and *Korthalsella japonica* (FIG. 9) on inhibiting productions of TNF-α caused by yellow dust, TSP, PM10 and PM2.5.
FIGS. 10 to 12 are chromatograms of ethanol extracts of *Citrus sunki* peel (FIG. 10), *Sceptridium ternatum* (or *Botrychium ternatum*) (FIG. 11) and *Korthalsella japonica* (FIG. 12), as analyzed by means of HPLC-UVD.
FIG. 13 is a graph indicating effects of 3 kinds of flavonoid glycoside separated from an ethanol extract of *Korthalsella japonica* on inhibiting a production of NO and a production of IL-8 and TNF-α by means of yellow dust and fine particulate.
FIG. 14 indicates experimental results of a cytokine array conducted to see whether inflammatory cytokines are produced or not according to types of yellow dust and fine particulate.

### Mode for Invention

Hereinafter, the present invention will be described in more detail through examples. The following examples are set forth just as the illustrative description of the present invention, but are not to be construed to limit the content of the present invention.

### Example 1. Preparing of Citrus sunki peel, Sceptridium ternatum (or Botrychium ternatum) and Korthalsella japonica extracts

500 ml of 70% ethanol (with purified water and ethanol mixed at a volume ratio of 30:70) was added to each 50 g of dry pulverized *Citrus sunki* peel, *Sceptridium ternatum* (or *Botrychium ternatum)* or *Korthalsella japonica,* after which resulting mixtures were shaken at room temperature for 20 hours to obtain extracts. These extracts were filtered and concentrated under reduced pressure to eliminate ethanol therefrom, such that resulting concentrates were freeze-dried to obtain ethanol extracts of *Citrus sunki* peel, *Sceptridium ternatum* (or *Botrychium ternatum*) and *Korthalsella japonica* at a yield of 17.9%, 18.3% and 15%, respectively.

### Example 2. Preparing of yellow dust and fine particulate extracts

### (1) Collecting of yellow dust and fine particulate

A Low Volume Air Sampler installed in Bucheon city, Gyeonggi-do province in March 2013 was used, and samples collected from the Sampler equipped with a 47 mm Teflon filter for 24 hours at a flow rate of 16.7 L per minute were used. The samples were collected separately with regard to yellow dust, TSP, PM10 and PM2.5 depending on filter types.

### (2) Preparing of yellow dust and fine particulate extracts

Each of distilled water, ethanol and chloroform was added to a filter cut in about 1 m² size, after which resulting filtrates were stirred for 12 hours to obtain extracts. The resulting extracts were separated, after which an extraction solvent was added again to extraction residues to obtain extracts again with ultrasonic waves for 4 hours. The extracts were combined together and concentrated under reduced pressure to evaporate and remove the extraction solvent therefrom, after which the samples extracted with distilled water and ethanol were dissolved again with respective solvents, while the sample extracted with chloroform was dissolved in DMSO.

### Experimental Example 1. Evaluation of the ability of yellow dust and fine particulate extracts to cause inflammation

Following experiments were performed in order to identify if the yellow dust, TSP, PM10 and PM2.5 extracts cause inflammation in cells.

### (1) Evaluation of caused NO production

NO, which is a pro-inflammatory mediator, is produced by means of various inflammatory stimuli and is known to cause continuous inflammatory responses and contribute to tissue damages. Thus, it was identified if the yellow dust and fine particulate extracts caused a production of NO. Raw 264.7 cells were seeded at 6 x 10⁴ cells/well into a 96-well plate, after which the resulting cells were cultured under a condition of 37°C and 5% CO□ for 24 hours. The resulting cells were treated with the yellow dust, TSP, PM10 or PM2.5 samples, which were diluted at each concentration, such that resulting treated cells were cultured again for 24 hours. After that, the resulting cell culture media were mixed with Griess reagent at a ratio of 1:1, after which their optical densities were measured at 540 nm by means of a Microplate Reader to measure a NO production. At that time, no cytotoxicity was detected from the Raw 264.7 cells of the samples for causing the NO production.

As shown in a following Table 1, it was identified that the distilled water extracts of yellow dust, TSP, PM10 and PM2.5 caused skin inflammation. When it comes to each of extraction solvents, it was shown that an extraction with distilled water caused the NO production most.

**[Table 1]**

| Sample | Concentration | NO production (%) | | | |
|---|---|---|---|---|---|
| | | Yellow dust | TSP | PM10 | PM2.5 |
| Control | | 100.0±2.1 | | | |
| Chloroform extract | 500x-dilution | 122.6±3.2 | 114.3±5.4 | 110.5±3.9 | 46.8±2.0 |
| | 100x-dilution | 83.9±2.2 | 86.3±2.8 | 79.4±2.7 | 74.3±12.4 |
| Ethanol extract | 500x-dilution | 126.0±8.5 | 130.4±3.0 | 123-9±1.0 | 134.2±19.1 |
| | 100x-dilution | 134.1±11.3 | 120.07±4.9 | 129.2±2.0 | 199.7±13.3 |
| Distilled extract | 500x-dilution | 192.7±1.3 | 225.9±5.7 | 136.6±2.3 | 196.2±5.1 |
| | 100x-dilution | 223.4±6.1 | 231.2±2.6 | 176.4±5.0 | 265.6±9.5 |

### (2) Evaluation of caused production of inflammatory cytokines IL-8, TNF-α. IL-1α, IL-2, IL-6, GRO-α and GM-CSF

IL-8, TNF-α, IL-1α, IL-2, IL-6, GRO-α and GM-CSF are representative of inflammatory cytokines, and the following method was performed in order to identify if the yellow dust and fine particulate extracts caused a production of TNF-α and IL-8. HaCaT cells were seeded at 2.5 x 10⁴ cells/well into a 96-well plate, after which the resulting cells were cultured under a condition of 37°C and 5% CO□ for 24 hours. The resulting cells were treated with the yellow dust, TSP, PM10 or PM2.5 samples, which were diluted at each concentration, such that resulting treated cells were cultured for 24 hours. After that, the resulting culture media were collected to evaluate their abilities to cause a production of cytokines by using an ELISA kit with regard to a fixed quantity of IL-1α, IL-2, IL-6, GRO-α, GM-CSF, IL-8 and TNF-α, wherein results thereof were shown in following Tables 2 to 4. At that time, no cytotoxicity was detected from the HaCaT cells of the samples for causing a production of IL-1α, IL-2, IL-6, GRO-α, GM-CSF, IL-8 and TNF-α.

As shown in following Tables 2 and 3, in case of IL-8, the yellow dust and PM2.5 only caused its production and the yellow dust caused such production most when being extracted with distilled water. In case of TNF-α, it was identified that the yellow dust, TSP and PM10, extracted with chloroform, as well as PM2.5, extracted with distilled water, caused its production most.

As shown in a following Table 4, it was identified that IL-1α, IL-2, IL-6 and GM-CSF were increased by means of the yellow dust, PM10, PM2.5 and TSP extracts, which were obtained by means of distilled water. In case of GRO-α, it was identified that only the distilled water extracts of the yellow dust and PM2.5 caused its production.

**[Table 2]**

| Sample | Concentration | IL-8 production (%) | | | |
|---|---|---|---|---|---|
| | | Yellow dust | TSP | PM10 | PM2.5 |
| Control | | 100.0±8.6 | | | |
| Chloroform extract | 500x-dilution | 155.8±3.6 | 58.3±5.0 | 45.8±9.0 | 88.0±1.9 |
| | 100x-dilution | 118.8±11.1 | 55.5±11.1 | 41.2±5.7 | 84.0±2.2 |
| Ethanol extract | 500x-dilution | 181.8±7.6 | 62.6±2.0 | 67.1±6.0 | 98.8±4.0 |
| | 100x-dilution | 148.2±9.1 | 71.3±4.0 | 53.0±3.0 | 89.5±0.4 |
| Distilled extract | 500x-dilution | 225.0±11.6 | 85.5±2.1 | 74.2±4.5 | 118.7±3.0 |
| | 100x-dilution | 234.8±13.2 | 85.0±9.4 | 84.8±7.7 | 127.4±5.5 |

**[Table 3]**

| Sample | Concentration | TNF-α production (%) | | | |
|---|---|---|---|---|---|
| | | Yellow dust | TSP | PM10 | PM2.5 |
| Control | | 100.0±8.4 | | | |
| Chloroform extract | 500x-dilution | 177.4±7.0 | 135.3±5.6 | 165.5±6.3 | 90.3±4.6 |
| | 100x-dilution | 217.8±8.8 | 162.7±4.5 | 176.2±10.5 | 75.3±6.2 |
| Ethanol extract | 500x-dilution | 137.8±3.0 | 115.1±1.8 | 129.2±1.7 | 91.9±5.0 |
| | 100x-dilution | 155.7±12.0 | 136.7±3.6 | 142.8±2.9 | 117.6±6.3 |
| Distilled extract | 500x-dilution | 98.1±5.0 | 146.7±4.0 | 124.1±1.3 | 115.9±6.1 |
| | 100x-dilution | 119.1±3.3 | 108.1±4.2 | 134.0±5.2 | 146.3±4.4 |

**[Table 4]**

| Sample | Concentration | Cytokine production (%) | | | | |
|---|---|---|---|---|---|---|
| | | 1L-1α | IL-2 | IL-6 | GRO-α | GM-CSF |
| Control | | 100.0±4.8 | 100.0±7.9 | 100.0±4.0 | 100.0±6.8 | 100.0±4.3 |
| Yellow dust | 100x-dilution | 160.6±1.8 | 150.1±9.7 | 558.0±11.3 | 173.4±2.0 | 435.2±16.0 |
| TSP | 100x-dilution | 123.2±5.1 | 190.2±12.2 | 283.7=14.3 | 87.5±5.2 | 122.7±6.0 |
| PM10 | 100x-dilution | 139.4±8.1 | 174.6±7.7 | 374.4±11.3 | 75.4±3.3 | 123.3±5.7 |
| PM2.5 | 100x-dilution | 166.9±4.2 | 155.7±5.7 | 140.1±7.1 | 120.2±2.8 | 121.4±2.5 |

### Experimental Example 2. Identification of the cytotoxicity of Citrus sunki peel, Sceptridium ternatum (or Botrychium ternatum) and Korthalsella japonica extracts

An experiment was performed by means of a following method in order to identify if the ethanol extracts of *Citrus sunki* peel, *Sceptridium ternatum* (or *Botrychium ternatum)* and *Korthalsellajaponica* have cytotoxicity. Raw 264.7 cells and HaCaT cells were seeded at 6.0 x 10⁴ cells/well and 2.5 x 10⁴ cells/well respectively, after which the resulting cells were cultured under a condition of 37°C and 5% CO□ for 24 hours. The resulting cultured cells were treated with the ethanol extracts of *Citrus sunki peel*, *Sceptridium ternatum* (or *Botrychium ternatum)* or *Korthalsella japonica* at a concentration of 50-1,600 *µ*g/ml, after which resulting treated cells were treated and reacted with a WST-1 solution in 24 hours later, such that their optical densities were measured at 450 nm by means of a Microplate Reader to identify a cell viability, wherein results of the ethanol extracts of *Citrus sunki* peel, *Sceptridium ternatum* (or *Botrychium ternatum*) and *Korthalsella japonica* were shown in following Tables 5 to 7.

As shown in following Tables 5 to 7, it was identified that the ethanol extracts of *Citrus sunki* peel, *Sceptridium ternatum* (or *Botrychium ternatum*) and *Korthalsella japonica* did not show cytotoxicity at 50-400 *µ*g/ml.

**[Table 5]**

| Sample concentration (*µ*g/ml) | | Raw 264.7 cell viability (%) | HaCaT cell viability (%) |
|---|---|---|---|
| Control | | 100.0±2.7 | 100.0±3.9 |
| *Citrus sunki* peel | 1,600 | 33.6±2.7 | 45.3±2.2 |
| | 800 | 75.6±0.7 | 88.1±1.5 |
| | 400 | 92.2±1.4 | 96.7±1.3 |
| | 200 | 98.0±3.5 | 95.8±4.1 |
| | 100 | 107.0±1.6 | 96.2±0.5 |
| | 50 | 99.9±3.4 | 92.5±0.8 |

**[Table 6]**

| Sample concentration (*µ*/ml) | | Raw 264.7 cell viability (%) | HaCaT cell viability (%) |
|---|---|---|---|
| Control | | 100.0±2.7 | 100.0±3.9 |
| *Sceptridium ternatum* (or *Botrychium ternatum*) | 1,600 | 34.6±1.4 | 44.3±4.1 |
| | 800 | 83.0±1.3 | 88.7±0.8 |
| | 400 | 93.4±1.2 | 101.4±3.3 |
| | 200 | 95.5±0.9 | 98.5±3.5 |
| | 100 | 99.2±2.3 | 97.5±3.3 |
| | 50 | 100.7±0.9 | 100.6±0.7 |

**[Table 7]**

| Sample concentration (*µ*/ml) | | Raw 264.7 cell viability (%) | HaCaT cell viability (%) |
|---|---|---|---|
| Control | | 100.0±2.7 | 100.0±3.9 |
| *Korthalsella japonica* | 1,600 | 24.7±0.4 | 29.0±1.9 |
| | 800 | 67.8±2.6 | 68.9±1.0 |
| | 400 | 94.7±2.1 | 93.9±1.6 |
| | 200 | 104.5±2.3 | 96.8±1.6 |
| | 100 | 103.2±0.9 | 101.4±2.1 |
| | 50 | 103.0±1.3 | 98.0±3.8 |

### Experimental Example 3. Evaluation of the ability of Citrus sunki peel, Sceptridium ternatum (or Botrychium ternatum) and Korthalsella japonica extracts to alleviate inflammation caused by yellow dust and fine particulate

### (1) Evaluation of the ability of ethanol extracts of Citrus sunki peel, Sceptridium ternatum (or Botrychium ternatum) and Korthalsella japonica to inhibit No production

In the above Experimental Example 1, it was identified that the yellow dust, TSP, PM10 and PM2.5 caused a production of NO in cells, and in result the distilled water extract for each sample caused the NO production most. Thus, the 100x diluted distilled water extracts of the yellow dust, TSP, PM10 and PM2.5 were used as a stimulus-causing agent, after which the *Citrus sunki* peel, *Sceptridium ternatum* (or *Botrychium ternatum*) and *Korthalsella japonica* extracts were treated at a concentration of 100-400 *µ*g/ml without cytotoxicity, such that their abilities to inhibit a NO production were evaluated by means of a following method. An extract of *Portulaca oleracea* L., generally known to soothe skin and have an excellent anti-inflammatory action was treated together as a positive control with regard to an effect of alleviating inflammation.

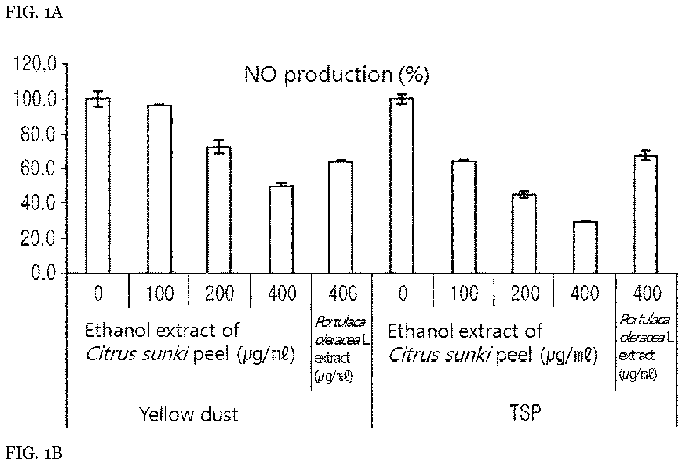

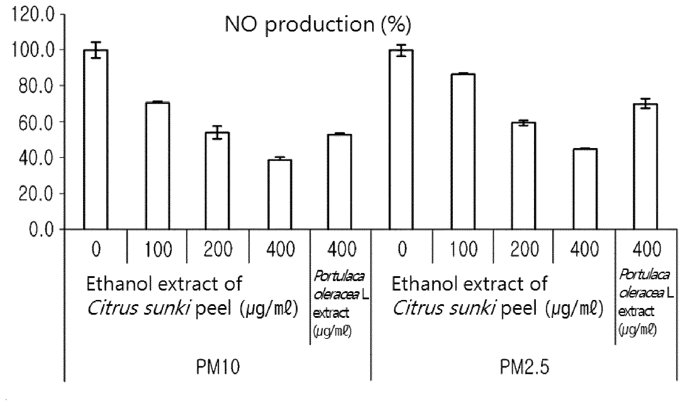

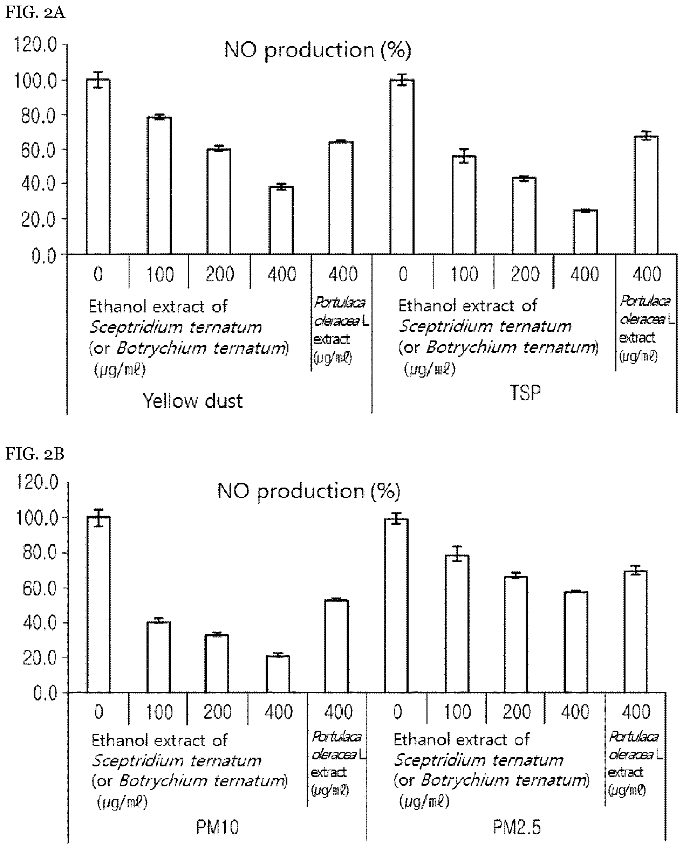

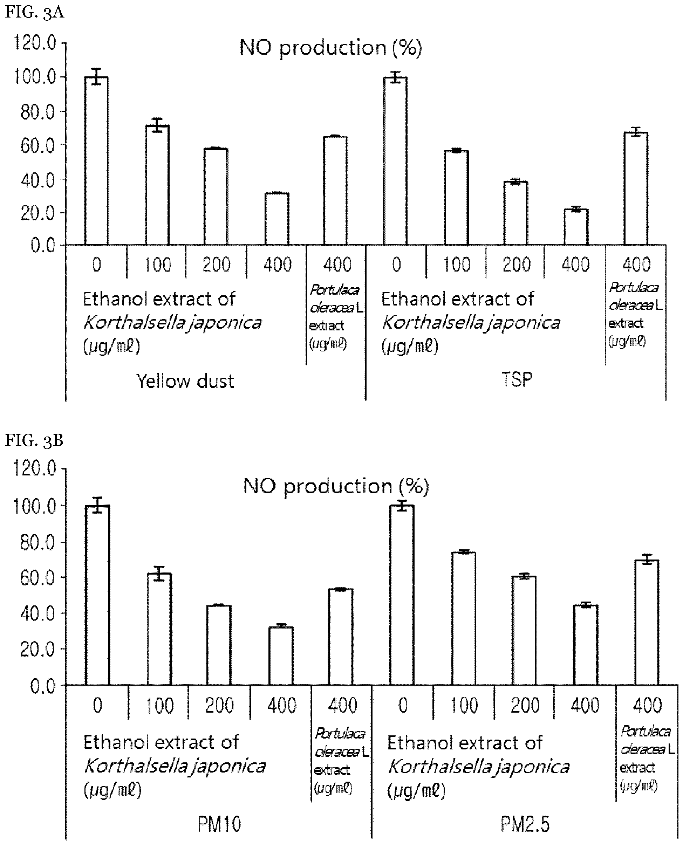

Raw 264.7 cells were seeded at 6.0 x 10⁴ cells/well into a 96-well plate, after which the resulting cells were cultured under a condition of 37°C and 5% CO□ for 24 hours. The resulting cultured cells were treated with the ethanol extracts of *Citrus sunki* peel, *Sceptridium ternatum* (or *Botrychium ternatum*) or *Korthalsella japonica* at a concentration of 100, 200 and 400 *µ*g/ml, after which resulting treated cells were treated with 100x diluted distilled water extracts of the yellow dust, TSP, PM10 or PM2.5, such that the resulting treated cells were cultured again for 24 hours. After that, the resulting cell culture media were mixed with Griess reagent at a ratio of 1:1, after which their optical densities were measured at 540 nm by means of a Microplate Reader to measure a NO production. When a control was the group treated only with the yellow dust, TSP, PM10 or PM2.5, which are air pollutants, the results of the ethanol extracts of *Citrus sunki* peel, *Sceptridium ternatum* (or *Botrychium ternatum*), *Korthalsella japonica* and *Portulaca oleracea* L. with regard to a degree of reduction in NO production amounts were shown in FIGS. 1 to 3.

As shown in FIGS. 1 to 3, the ethanol extracts of *Citrus sunki* peel, *Sceptridium ternatum* (or *Botrychium ternatum*) and *Korthalsella japonica* showed an effect of inhibiting a NO production in a concentration-dependent way and showed a more excellent effect than the *Portulaca oleracea* L. extract at the same concentration. Based on such results, it was identified that the ethanol extracts of *Citrus sunki* peel, *Sceptridium ternatum* (or *Botrychium ternatum*) and *Korthalsella japonica* could effectively inhibit the pro-inflammatory mediator NO.

Also, when it comes to an inhibitory effect on the NO production according to each of air pollutants at the same concentration, it was identified that the ethanol extract of *Korthalsella japonica* had a relatively more excellent effect on the yellow dust and TSP, the ethanol extract of *Sceptridium ternatum* (or *Botrychium ternatum*) had a relatively more excellent effect on the PM10, and the ethanol extract of *Korthalsella japonica* had a relatively more excellent effect on the PM2.5 than other extracts.

### (2) Evaluation of the ability of ethanol extracts of Citrus sunki peel, Sceptridium ternatum (or Botrychium ternatum) and Korthalsella japonica to inhibit a production of cytokines

As shown in the above Experimental Example 1, in case of IL-8 and GRO-α, the distilled water extracts of yellow dust and PM2.5 caused their production. In case of the chloroform extracts of yellow dust, TSP and PM10 and the distilled water extract of PM2.5 caused its production. Also, in case of IL-1α, IL-2, IL-6 and GM-CSF, the distilled water extracts of yellow dust, PM10, PM2.5 and TSP caused their production. Thus, the 100x-diluted distilled water extracts of yellow dust and PM2.5 were used as a stimulus-causing agent for IL-8 and GRO-α. The 100x-diluted chloroform extracts of yellow dust, TSP and PM10 and the 100x-diluted distilled water extracts of PM2.5 were used as a stimulus-causing agent for TNF-α. The 100x-diluted distilled water extracts of yellow water, PM10, PM2.5 and TSP were used as a stimulus-causing agent for IL-1α, IL-2, IL-6 and GM-CSF, which were the rest of cytokines. The above ethanol extracts of *Citrus sunki* peel, *Sceptridium ternatum* (or *Botrychium ternatum*) and *Korthalsella japonica* were treated at 100-400 *µ*g/ml, such that their abilities to inhibit a production of inflammatory cytokines were evaluated as follows.

HaCaT cells were seeded at 2.5 x 10⁴ cells/well into a 96-well plate, after which the resulting cells were cultured under a condition of 37°C and 5% COD for 24 hours. Each well plates was treated with the above stimulus-causing agent, after which the resulting cells were cultured again for 24 hours. After that, the resulting culture media were collected to quantify their cytokines by using an ELISA kit with regard to a fixed quantity of IL-1α, IL-2, IL-6, IL-8, GRO-α, GM-CSF and TNF-α.

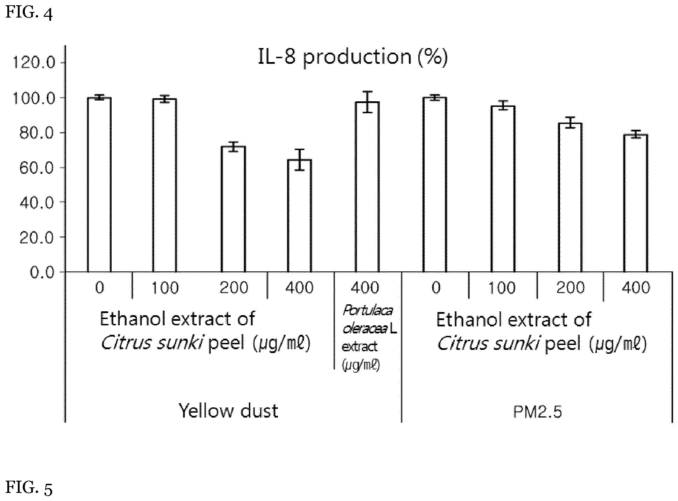

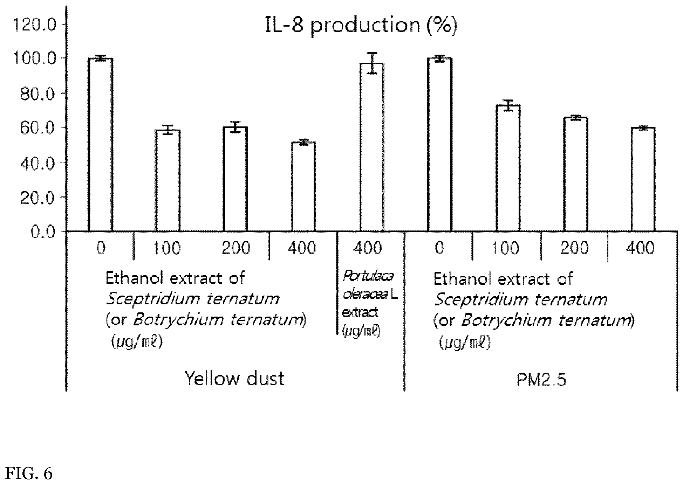

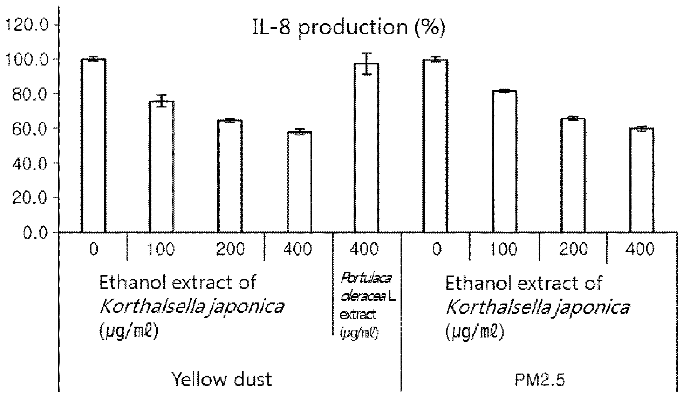

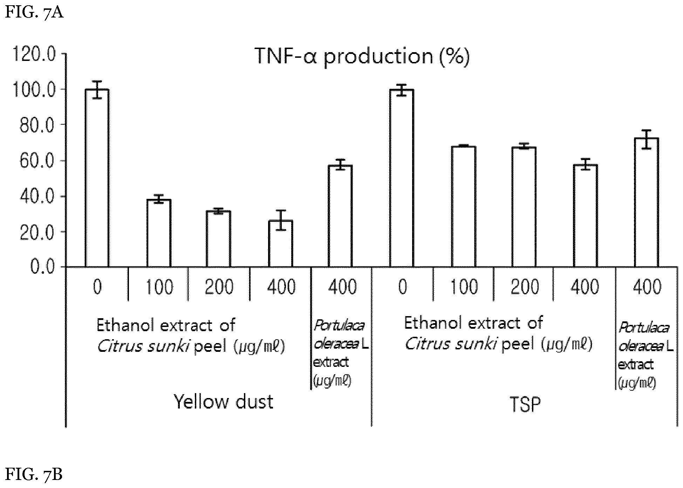

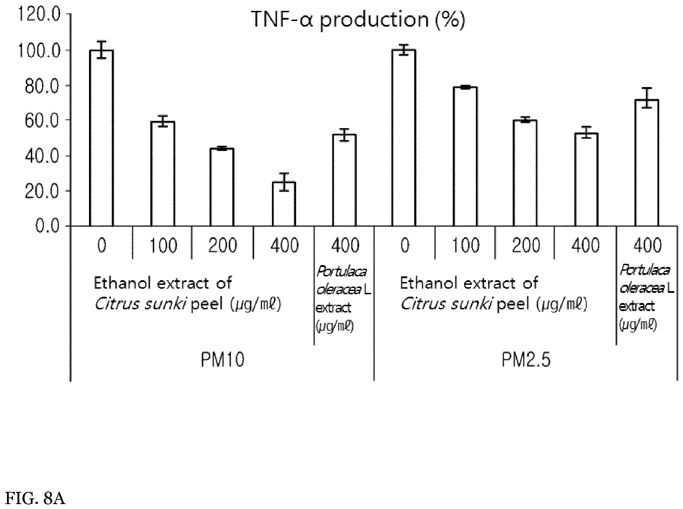

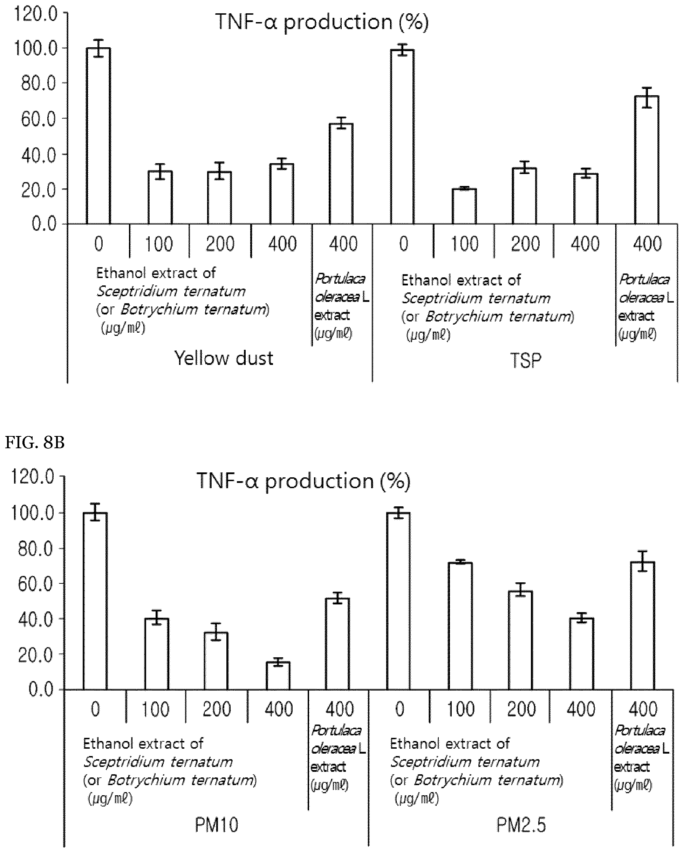

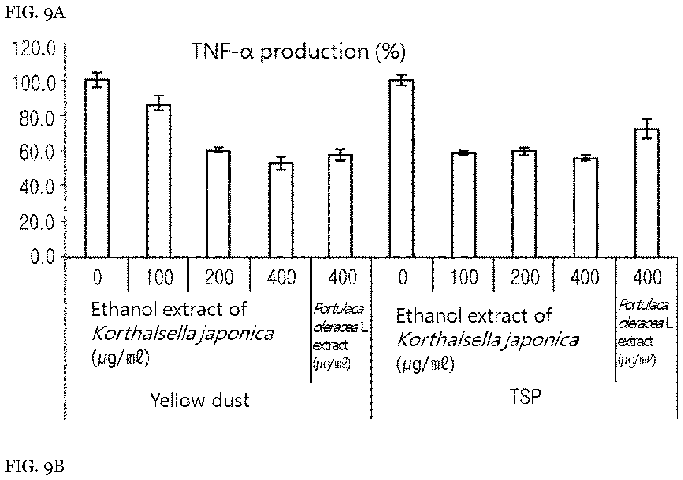

In case of IL-8 and an extract of *Portulaca oleracea* L., generally known to soothe skin and have an excellent anti-inflammatory action, was treated together as a positive control with regard to an effect of alleviating inflammation. When a control was the group treated only with the yellow dust, TSP, PM10 or PM2.5, which are air pollutants, the results thereof with regard to a degree of reduction in IL-8 and TNF-α production amounts were shown in FIGS 4 to 9.

In case of IL-1α, IL-2, IL-6, GRO-α and GM-CSF, the production amounts of IL-1α, IL-2, IL-6, GRO-α and GM-CSF were measured with regard to a untreated group, a group treated only with the yellow dust, TSP, PM10 or PM2.5, which are air pollutants, and a group treated with ethanol extracts of *Citrus sunki* peel, *Sceptridium ternatum* (or *Botrychium ternatum*) or *Korthalsellajaponica* at a concentration of 100, 200 and 400 *µ*g/ml, wherein results thereof were shown in following Tables 8 to 12.

As shown in FIGS. 4 to 9, the ethanol extracts of *Citrus sunki* peel, *Sceptridium ternatum* (or *Botrychium ternatum*) and *Korthalsella japonica* showed an effect of inhibiting a production of IL-8 and TNF-α in a concentration-dependent way and showed a more excellent effect than the *Portulaca oleracea* L. extract at the same concentration. Also, as shown in following Tables 8 to 12, it was identified that the production amounts of IL-1α, IL-2, IL-8 and GRO-α, which were increased by means of air pollutants, were also inhibited by the ethanol extracts of *Sceptridium ternatum* (or *Botrychium ternatum*), *Korthalsella japonica* and *Citrus sunki* peel in a concentration-dependent way. Based on such results, it was identified that the ethanol extracts of *Sceptridium ternatum* (or *Botrychium ternatum*) and *Korthalsella japonica* could effectively inhibit the inflammatory cytokines IL-1α, IL-2, IL-6, IL-8, GRO-α, GM-CSF and TNF-α. In case of the *Citrus sunki* peel, it failed to produce results due to some problem in process of the IL-6 experiment and it was identified that it could inhibit the inflammatory cytokines IL-1α, IL-2, IL-8, GRO-α, GM-CSF and TNF-α except IL-6.

When it comes to an inhibitory effect on the IL-8 production according to each of the air pollutants at the same concentration, it was identified that the ethanol extract of *Sceptridium ternatum* (or *Botrychium ternatum*) was relatively more excellent with regard to the yellow dust, while the ethanol extracts of *Sceptridium ternatum* (or *Botrychium ternatum*) and *Korthalsella japonica* were relatively more excellent with regard to the PM2.5 than other extracts. When it comes to an inhibitory effect on the TNF-α production according to each of the air pollutants at the same concentration, it was identified that the ethanol extract of *Citrus sunki* peel was relatively more excellent with regard to the yellow dust, while the ethanol extract of *Sceptridium ternatum* (or *Botrychium ternatum*) was relatively more excellent with regard to the TSP, PM10 and PM2.5 than other extracts. When it comes to an inhibitory effect on the IL-1α production according to each of the air pollutants at the same concentration, it was identified that the ethanol extract of *Sceptridium ternatum* (or *Botrychium ternatum*) was relatively more excellent with regard to the PM2.5 and TSP than other extracts. When it comes to an inhibitory effect on the IL-2 production according to each of the air pollutants at the same concentration, it was identified that the ethanol extracts of *Sceptridium ternatum* (or *Botrychium ternatum*) and *Citrus sunki* peel were relatively more excellent with regard to the yellow dust and PM10, while the ethanol extract of *Sceptridium ternatum* (or *Botrychium ternatum*) was relatively more excellent with regard to the PM2.5 and TSP than other extracts. When it comes to an inhibitory effect on the production of IL-6 and GRO-α for all the air pollutants at the same concentration, it was identified that the ethanol extract of *Sceptridium ternatum* (or *Botrychium ternatum*) was relatively more excellent with regard to other extracts. When it comes to an inhibitory effect on the IL-6 production, the ethanol extract of *Sceptridium ternatum* (or *Botrychium ternatum*) had a relatively more excellent effect than other extracts. When it comes to an inhibitory effect on the GM-CSF production for all the air pollutants at the same concentration, the ethanol extract of *Citrus sunki* peel had a relatively more excellent effect than other extracts.

**[Table 8]**

| Sample concentration (*µ*g/ml) | | IL-1α production (%) | | | |
|---|---|---|---|---|---|
| | | Yellow dust | TSP | PM10 | PM2.5 |
| Control | | 100.0±5.0 | | | |
| Group treated with air pollutants | | 163.6±8.9 | 157.4±10.3 | 124.6±1.8 | 168.3±5.6 |
| *Citrus sunki* peel | 100 | 145.4±5.9 | 156.8±6.0 | 100.2±2.4 | 138.0±6.3 |
| | 200 | 132.3±4.1 | 127.2±5.7 | 97.8±1.6 | 132.9±3.0 |
| | 400 | 128.7±3.1 | 116.2±2.6 | 94.4±1.9 | 125.0±0.6 |
| *Sceptridium ternatum* (or *Botrychium ternatum*) | 100 | 118.9±3.8 | 120.6±4.2 | 96.7±4.2 | 110.8±3.5 |
| | 200 | 112.1±2.1 | 111.4±0.9 | 91.3±3.0 | 99.5±1.6 |
| | 400 | 100.1±4.1 | 97.8±1.3 | 83.1±1.2 | 92.5±4.6 |
| *Korthalsella japonica* | 100 | 150.7±6.9 | 166.7±6.0 | 114.8±1.2 | 143.9±9.1 |
| | 200 | 129.0±5.6 | 157.6±4.7 | 103.7±2.3 | 133.9±4.8 |
| | 400 | 112.4±3.7 | 143.3±2.8 | 97.4±4.4 | 120.2±2.2 |

**[Table 9]**

| Sample concentration (*µ*g/ml) | | IL-2 production (%) | | | |
|---|---|---|---|---|---|
| | | Yellow dust | TSP | PM10 | PM2.5 |
| Control | | 100.0±0.6 | | | |
| Group treated with air pollutants | | 146.5±4.9 | 152.9±1.7 | 122.9±5.2 | 126.5±2.7 |
| *Citrus sunki* peel | 100 | 94.6±4.2 | 112.7±4.1 | 91.9±1.5 | 100.0±1.9 |
| | 200 | 86.3±2.9 | 96.8±3.5 | 77.3±3.4 | 89.4±1.2 |
| | 400 | 78.1±5.2 | 91.6±6.1 | 71.8±2.0 | 85.1±2.5 |
| *Sceptridium ternatum* (or *Botrychium ternatum*) | 100 | 106.7±3.8 | 112.5±3.1 | 94.0±5.3 | 104.6±4.0 |
| | 200 | 83.8±1.5 | 103.6±2.4 | 74.2±3.2 | 83.6±3.0 |
| | 400 | 71.6±2.0 | 81.6±4.2 | 65.7±2.3 | 72.0±2.4 |
| *Korthalsella japonica* | 100 | 137.6±1.3 | 150.2±4.9 | 121.2±4.5 | 132.1±2.5 |
| | 200 | 107.3±5.4 | 135.5±2.4 | 98.6±5.1 | 124.2±3.3 |
| | 400 | 99.1±2.5 | 116.6±5.9 | 89.9±5.5 | 109.4±3.1 |

**[Table 10]**

| Sample concentration (*µ*g/ml) | | IL-6 production (%) | | | |
|---|---|---|---|---|---|
| | | Yellow dust | TSP | PM10 | PM2.5 |
| Control | | 100.0±6.9 | | | |
| Group treated with air pollutants | | 344.6±9.6 | 244.0±9.3 | 299.8±12.0 | 131.9±5.6 |
| *Sceptridium* | 100 | 208.5±7.5 | 215.6±10.3 | 198.2±11.0 | 227.4±14.5 |
| *ternatum* (or *Botrychium ternatum*) | 200 | 177.8±10.0 | 202.3±6.2 | 166.8±5.5 | 198.2±16.3 |
| | 400 | 153.3±7.7 | 154.7±3.3 | 151.5±3.0 | 182.8±12.4 |
| *Korthalsella japonica* | 100 | 287.3±9.9 | 256.9±13.8 | 315.8±2.4 | 251.5±7.3 |
| | 200 | 228.7±10.0 | 229.4±5.8 | 265.5±2.8 | 237.7±11.4 |
| | 400 | 211.2±4.1 | 190.5±11.9 | 238.8±7.9 | 220.1±1.8 |

**[Table 11]**

| Sample concentration (*µ*g/ml) | | GRO-α production (%) | |
|---|---|---|---|
| | | Yellow dust | PM2.5 |
| Control | | 100.0±1.5 | |
| Group treated with air pollutants | | 188.7±5.5 | 151.2±4.7 |
| *Citrus sunki* peel | 100 | 91.7±3.5 | 73.8±4.2 |
| | 200 | 76.8±4.4 | 62.5±3.0 |
| | 400 | 30.6±0.9 | 42.1±2.2 |
| *Sceptridium ternatum* (or *Botrychium ternatum*) | 100 | 56.3±2.3 | 84.6±6.7 |
| | 200 | 30.9±1.5 | 45.9±2.5 |
| | 400 | 13.1±0.8 | 23.2±1.1 |
| *Korthalsella japonica* | 100 | 58.1±5.1 | 48.9±1.7 |
| | 200 | 39.7±0.7 | 29.2±1.9 |
| | 400 | 17.5±0.3 | 24.2±2.4 |

**[Table 12]**

| Sample concentration (*µ*g/ml) | | GM-CSF production (%) | | | |
|---|---|---|---|---|---|
| | | Yellow dust | TSP | PM10 | PM2.5 |
| Control | | 100.0±1.5 | | | |
| Group treated with air pollutants | | 288.3±11.3 | 224.3±2.0 | 223.8±8.5 | 187.5±9.7 |
| *Citrus sunki* peel | 100 | 104.1±8.0 | 60.1±5.3 | 48.0±0.6 | 59.4±2.3 |
| | 200 | 83.0±5.8 | 54.8±2.2 | 45·8±1.2 | 53.7±1.3 |
| | 400 | 69.3±7.0 | 53.6±2.4 | 47.1±1.7 | 51.1±1.9 |
| *Sceptridium ternatum* (or *Botrychium ternatum*) | 100 | 249.8±11.2 | 172.4±10.8 | 254.5±10.8 | 213.3±6.3 |
| | 200 | 214.2±10.2 | 160.9±10.9 | 197.9±12.18 | 146.2±1.6 |
| | 400 | 180.3±11.9 | 92.3±5.3 | 151.9±2.2 | 108.5±9.9 |
| *Korthalsella japonica* | 100 | 417.7±14.8 | 382.0±18.2 | 400.9±11.2 | 331.5±14.7 |
| | 200 | 328.3±9.6 | 348.7±4.3 | 312.0±14.5 | 314.4±10.3 |
| | 400 | 312.1±3.8 | 221.7±13.7 | 269.6±11.8 | 269.0±5.9 |

### Experimental Example 4. Evaluation of the ability of mixture of Citrus sunki peel, Sceptridium ternatum (or Botrychium ternatum) and Korthalsella japonica extracts to alleviate inflammation caused by yellow dust and fine particulate

As seen in the above Experimental Example 3, the effects of *Citrus sunki* peel, *Sceptridium ternatum* (or *Botrychium ternatum*) and *Korthalsella japonica* extracts are not equal to each other depending on air pollutants and inflammatory cytokines. Thus, it was thought that using a mixture of *Citrus sunki* peel, *Sceptridium ternatum* (or *Botrychium ternatum*) and *Korthalsella japonica* extracts might exhibit an effect on inflammatory cytokines produced and caused by all the air pollutants, so an experiment was performed on its ability to inhibit a production of NO and inhibit a production of inflammatory cytokines. Also, the experiment was performed on each extract of the same amount in order to identify a synergy effect of the mixture.

### (1) Evaluation of the ability of mixture of ethanol extracts of Citrus sunki peel, Sceptridium ternatum (or Botrychium ternatum) and Korthalsella japonica to inhibit NO production

The same 100x-diluted distilled water extract as used in the above Experimental Example 3(1) was used as a stimulus-causing agent for NO. Mixtures used herein were prepared in such a way that the ethanol extracts of *Sceptridium ternatum* (or *Botrychium ternatum*), *Korthalsella japonica* and *Citrus sunki* peel were mixed at the same ratio (hereinafter, a 1:1:1 mixture) or that the ethanol extract of *Citrus sunki* peel was mixed twice as much as the ethanol extract of *Sceptridium ternatum* (or *Botrychium ternatum*) and the ethanol extract of *Korthalsella japonica* (hereinafter, a 2:1:1 mixture), respectively. The ethanol extracts of *Sceptridium ternatum* (or *Botrychium ternatum*), *Korthalsella japonica* and *Citrus sunki* peel and mixtures thereof were treated at a concentration of 52.5 *µ*g/ml, 105 *µ*g/ml and 210 *µ*g/ml, respectively, wherein their abilities to inhibit the NO production were evaluated by means of a following method.

HaCaT cells were seeded at 2.5 x 10⁴ cells/well into a 96-well plate, after which the resulting cells were cultured under a condition of 37°C and 5% CO□ for 24 hours. The resulting cultured cells were treated with the ethanol extracts of *Sceptridium ternatum* (or *Botrychium ternatum), Korthalsella japonica* or *Citrus sunki* peel, or mixtures thereof at a concentration of 52.5, 105 and 210 *µ*g/ml, after which resulting treated cells were treated with 100x-diluted distilled water extracts of yellow dust, PM10, PM2.5 or TSP, such that resulting treated cells were cultured again for 24 hours. After that, the resulting culture media were collected to quantify a NO amount by using a NO assay, wherein results thereof with regard to the mixtures of ethanol extracts of *Citrus sunki* peel, *Sceptridium ternatum* (or *Botrychium ternatum)* and *Korthalsella japonica* were shown in a following Table 13.

As shown in a following Table 13, the mixture of ethanol extracts of *Citrus sunki* peel, *Sceptridium ternatum* (or *Botrychium ternatum)* and *Korthalsella japonica* showed an inhibitory effect on a NO production in a concentration-dependent way, and it was identified that the existing ethanol extracts of *Sceptridium ternatum* (or *Botrychium ternatum*), *Korthalsella japonica* and *Citrus sunki* peel as well as mixtures thereof had the same tendency to inhibit the NO production. At the same concentration, the 2:1:1 mixture tended to show a more excellent effect than the 1:1:1 mixture. Also, in case of the inhibitory effect on the NO production, it was identified that the mixtures had a more excellent effect than single extracts with regard to TSP at the same concentration.

**[Table 13]**

| Sample concentration (ug/ml) | | NO production (%) | | | |
|---|---|---|---|---|---|
| | | Yellow dust | TSP | PM10 | PM2.5 |
| Control | | 100.0±5.4 | 100.0±1.0 | 100.0±3.3 | 100.0±3.2 |
| Group treated with air pollutants | | 136.5±1.8 | 151.4±2.0 | 147.1±3.9 | 147.1±1.5 |
| *Citrus sunki* peel | 52.5 | 124.9±2.2 | 111.4±5.5 | 118.9±4.4 | 150.9±4.8 |
| | 105 | 113.3±5.0 | 100.1±1.6 | 99.9±3.7 | 127.2±6.4 |
| | 210 | 104.2±3.3 | 83.5±2.7 | 80.1±3.1 | 106.7±2.6 |
| *Sceptridium ternatum* (or *Botrychium ternatum*) | 52.5 | 128.6±0.9 | 118.0±2.9 | 125.6±6.0 | 158.3±1.7 |
| | 105 | 116.6±1.8 | 98.9±2.2 | 117.2±6.5 | 131.6±8.2 |
| | 210 | 104.0±1.6 | 79.2±3.2 | 94.3±2.1 | 108.6±2.9 |
| *Korthalsella japonica* | 52.5 | 123.4±3.3 | 117.2±3.4 | 116.3±6.8 | 134.0±5.1 |
| | 105 | 104.3±5.2 | 99.4±2.3 | 91.5±3.5 | 112.7±4.3 |
| | 210 | 90.2±6.5 | 79.9±3.2 | 71.5±2.7 | 95.7±5.7 |
| Mixture (1:1:1) | 52.5 | 125.9±5.2 | 126.3±2.4 | 126.0±2.1 | 146.2±2.8 |
| | 105 | 110.7±2.4 | 86.8±2.2 | 114.5±3.5 | 126.9±6.1 |
| | 210 | 95.4±4.6 | 52.8±1.1 | 96.3±4.2 | 111.1±6.3 |
| Mixture (1:1:2) | 52.5 | 117.8±2.6 | 103.1±1.5 | 125.0±1.3 | 152.2±3.9 |
| | 105 | 107.7±5.4 | 83.6±0.8 | 99.8±2.5 | 122.7±6.5 |
| | 210 | 94.8±6.0 | 58.5±1.9 | 86.1±2.2 | 100.1±3.4 |

### (2) Evaluation of the ability of mixture of ethanol extracts of Citrus sunki peel, Sceptridium ternatum (or Botrychium ternatum) and Korthalsella japonica to inhibit the production of cytokines

As shown in the above Experimental Example 1, the 100x-diluted distilled water extracts of yellow dust and PM2.5 were used as a stimulus-causing agent for IL-8 and GRO-α, the 100x-diluted chloroform extracts of yellow dust, TSP and PM10 and the 100x-diluted distilled water extract of PM2.5 were used as a stimulus-causing agent for TNF-α, and the distilled water extracts of yellow dust, PM10, PM2.5 and TSP were used as a stimulus-causing agent for IL-1α, IL-2 and GM-CSF. A 1:1:1 mixture and a 2:1:1 mixture of the ethanol extracts of *Sceptridium ternatum* (or *Botrychium ternatum), Korthalsella japonica* and *Citrus sunki* peel were prepared and used respectively. The ethanol extracts of *Sceptridium ternatum* (or *Botrychium ternatum*), *Korthalsella japonica* and *Citrus sunki* peel as well as mixtures thereof were treated at a concentration of 52.5 *µ*g/ml, 105 *µ*g/ml and 210 *µ*g/ml respectively, such that their abilities to inhibit the production of inflammatory cytokines were evaluated as follows.

The HaCaT cells were seeded at 2.5 x 10⁴ cells/well into a 96-well plate, after which the resulting cells were cultured under a condition of 37°C and 5% CO□ for 24 hours. The ethanol extracts of *Citrus sunki* peel, *Sceptridium ternatum* (or *Botrychium ternatum*) or *Korthalsellajaponica,* or mixtures thereof were treated at a concentration of 52.5, 105 and 210 *µ*g/ml respectively, and each well plates was treated with the above stimulus-causing agent, after which resulting treated cells were cultured again for 24 hours. After that, the resulting culture media were collected to quantify their cytokines by using an ELISA kit with regard to a fixed quantity of IL-1α, IL-2, IL-6, IL-8, GRO-α, GM-CSF and TNF-α, wherein results thereof with regard to the ethanol extracts of *Citrus sunki* peel, *Sceptridium ternatum* (or *Botrychium ternatum)* and *Korthalsella japonica* as well as mixtures thereof were shown in following Tables 14 to 19.

As shown in following Tables 14 to 19, the mixtures of ethanol extracts of *Citrus sunki* peel, *Sceptridium ternatum* (or *Botrychium ternatum)* and *Korthalsellajaponica* showed an inhibitory effect in a concentration-dependent way on the production of IL-1α, IL-2, IL-8, GRO-α, GM-CSF and TNF-α except IL-6, which is largely influenced by the ethanol extract of *Citrus sunki* peel, and it was identified that single ethanol extracts of *Citrus sunki* peel, *Sceptridium ternatum* (or *Botrychium ternatum)* and *Korthalsella japonica* as well as mixtures thereof had the same tendency to inhibit the production of cytokines. The 2:1:1 mixture tended to show a more excellent effect than the 1:1:1 mixture at the same concentration. Also, it was identified that according to each of air pollutants, the mixtures had a relatively more excellent effect on the yellow dust for IL-1α, PM10 for TNF-α, IL-2 and GM-CSF and TSP for IL-1α and IL-2 than the single extracts at the same concentration.

**[Table 14]**

| Sample concentration (*µ*g/ml) | | IL-1α production (%) | | | |
|---|---|---|---|---|---|
| | | Yellow dust | TSP | PM10 | PM2.5 |
| Control | | 100.0±4.0 | 100.0±4.0 | 100.0±4.0 | 100.0±4.0 |
| Group treated with air pollutants | | 144.8±3.0 | 141.0±3.4 | 128.5±3.8 | 141.6±6.1 |
| *Citrus sunki* peel | 52.5 | 109.6±2.1 | 94.6±3.0 | 79.7±3.5 | 97.1±3.5 |
| | 105 | 96.1±2.6 | 81.6±1.6 | 73.8±3.4 | 80.1±0.5 |
| | 210 | 83.8±4.4 | 73.2±4.0 | 67.2±3.1 | 72.4±1.5 |
| *Sceptridium ternatum* (or *Botrychium ternatum*) | 52.5 | 93.5±1.9 | 87.5± 2.9 | 79.7±1.2 | 76.4±1.8 |
| | 105 | 84.3±0.8 | 67.9±0.7 | 68.6±1.2 | 70.6±2.1 |
| | 210 | 80.9±1.3 | 62.2± 3.0 | 62.9±3.0 | 60.1±1.2 |
| *Korthalsella japonica* | 52.5 | 109.1±2.3 | 97.5±1.5 | 74.3±3.5 | 96.2±1.5 |
| | 105 | 95.0±1.2 | 83.6±2.9 | 70.4±2.2 | 77.4±1.8 |
| | 210 | 84.0±3.1 | 73.4±3.0 | 62.6±1.6 | 67.1±0.9 |
| Mixture (1:1:1) | 52.5 | 95.7±1.4 | 73.9± 1.0 | 79.8±3.2 | 73.5±3.6 |
| | 105 | 87.6±2.8 | 64.3±2.8 | 73.7±0.8 | 65.2±0.9 |
| | 210 | 77.4±3.5 | 59.5±1.9 | 66.7±1.0 | 58.8±1.5 |
| Mixture (1:1:2) | 52.5 | 82.4±1.5 | 73.4±1.3 | 71.8±2.3 | 71.3±1.2 |
| | 105 | 77.8±2.9 | 67.8±1.2 | 68.1±2.0 | 66.7±0.5 |
| | 210 | 70.2±1.5 | 62.9±0.4 | 60.7±2.9 | 63.5±2.7 |

**[Table 15]**

| Sample concentration (*µ*g/ml) | | IL-2 production (%) | | | |
|---|---|---|---|---|---|
| | | Yellow dust | TSP | PM10 | PM2.5 |
| Control | | 100.0±6.9 | 100.00±6.93 | 100.0±6.9 | 100.0±6.93 |
| Group treated with air pollutants | | 178.5±7.9 | 178.22±3.49 | 134.4±6.2 | 139.0±6.52 |
| *Citrus sunki* peel | 52.5 | 108.6±2.6 | 159.3±5.7 | 115.3±1.2 | 110.7±5.5 |
| | 105 | 98.3±5.2 | 137.9±2.6 | 95.0±5.6 | 82.5±5.7 |
| | 210 | 75.9±6.7 | 105.6±4.6 | 75.2±3.3 | 57.0±2.7 |
| *Sceptridium ternatum* (or *Botrychium ternatum*) | 52.5 | 105.2±1.4 | 137.41±5.33 | 104.1±1.7 | 90.3±5.21 |
| | 105 | 80.0±2.7 | 105.75±5.87 | 82.6±3.2 | 67.1±1.93 |
| | 210 | 65.0±5.0 | 83.26±9.52 | 64.5±2.3 | 49.7±3.30 |
| *Korthalsella japonica* | 52.5 | 140.0±2.6 | 158.04±5.03 | 130.8±6.9 | 120.5±2.31 |
| | 105 | 109.7±2.8 | 135.0±6.5 | 116.2±3.4 | 88.9±1.4 |
| | 210 | 93.8±5.8 | 102.0±4.5 | 91.0±6.1 | 77.0±4.1 |
| Mixture (1:1:1) | 52.5 | 100.9±4.5 | 132.8±2.2 | 77.7±5.1 | 98.6±5.2 |
| | 105 | 82.8±7.9 | 102.5±2.0 | 66.8±2.5 | 72.5±1.0 |
| | 210 | 61.5±5.0 | 75.3±5.3 | 48.9±4.4 | 46.4±3.9 |
| Mixture (1:1:2) | 52.5 | 98.6±3.4 | 121.6±0.9 | 75.6±4.3 | 100.5±3.6 |
| | 105 | 79.3±6.9 | 105.2±6.9 | 65.6±2.9 | 71.3±7.4 |
| | 210 | 66.5±3.6 | 64.8±3.2 | 46.0±6.2 | 33.2±4.3 |

**[Table 16]**

| Sample concentration (*µ*g/ml) | | IL-8 production (%) | |
|---|---|---|---|
| | | Yellow dust | PM2.5 |
| Control | | 100.0±3.6 | 100.0±3.4 |
| Group treated with air pollutants | | 133.0±3.3 | 133.5±3.7 |
| *Citrus sunki* peel | 52.5 | 80.5±4.7 | 67.0±5.3 |
| | 105 | 67.7±3.0 | 75.3±3.8 |
| | 210 | 58.0±1.5 | 87.1±3.8 |
| *Sceptridium ternatum* (or *Botrychium ternatum*) | 52.5 | 81.1±6.6 | 66.5±1.7 |
| | 105 | 68.0±4.5 | 85.8±3.7 |
| | 210 | 58.9±4.4 | 92.9±3.4 |
| *Korthalsella japonica* | 52.5 | 92.8±7.6 | 60.6±2.3 |
| | 105 | 77.8±5.0 | 80.5±2.1 |
| | 210 | 47.9±3.4 | 100.1±3.1 |
| Mixture (1:1:1) | 52.5 | 73.7±3.5 | 62.5±2.3 |
| | 105 | 60.1±1.3 | 79.2±2.8 |
| | 210 | 46.1±1.7 | 91.2±2.9 |
| Mixture (1:1:2) | 52.5 | 83.3±3.2 | 54.0±0.8 |
| | 105 | 72.4±2.6 | 77.1±2.6 |
| | 210 | 58.6±2.4 | 100.9±3.7 |

**[Table 17]**

| Sample concentration (*µ*g/ml) | | TNF-α production (%) | | | |
|---|---|---|---|---|---|
| | | Yellow dust | TSP | PM10 | PM2.5 |
| Control | | 100.0±6.2 | 100.0±4.9 | 100.0±3.4 | 100.0±4.9 |
| Group treated with air pollutants | | 161.6±4.7 | 140.8±0.7 | 149.8±8.2 | 171.9±7.2 |
| *Citrus sunki* peel | 52.5 | 120.0±2.3 | 126.5±5.5 | 113.4±7.9 | 154.9±7.4 |
| | 105 | 110.9±7.8 | 110.4±4.6 | 78.4±1.6 | 116.2±12.7 |
| | 210 | 81.8±4.7 | 96.7±4.3 | 52.0±5.7 | 89.1±7.9 |
| *Sceptridium ternatum* (or *Botrychium ternatum*) | 52.5 | 132.1±3.1 | 123.0±6.0 | 119.8±6.1 | 146.9±11.4 |
| | 105 | 100.3±6.2 | 106.8±3.4 | 93.2±5.8 | 95.6±6.3 |
| | 210 | 82.1±1.9 | 90.1±2.9 | 60.5±1.3 | 71.7±2.6 |
| *Korthalsella japonica* | 52.5 | 116.9±5.7 | 112.1±2.3 | 133.8±2.2 | 143.6±5.2 |
| | 105 | 88.6±6.1 | 98.6±4.9 | 101.1±8.9 | 116.7±9.9 |
| | 210 | 73.5±4.9 | 91.6±2.9 | 73.0±5.0 | 95.4±7.1 |
| Mixture (1:1:1) | 52.5 | 108.5±4.9 | 121.0±5.7 | 125.6±4.0 | 130.4±3.4 |
| | 105 | 91.9±8.4 | 96.0±0.9 | 71.0±3.0 | 105.2±2.3 |
| | 210 | 72.7±6.5 | 80.0±4.0 | 39.4±4.6 | 76.6±5.3 |
| Mixture (1:1:2) | 52.5 | 115.0±4.5 | 128.5±1.6 | 117.0±2.9 | 140.6±6.1 |
| | 105 | 97.5±3.1 | 111.7±3.3 | 75.7±2.9 | 115.1±7.6 |
| | 210 | 69.1±4.4 | 93.2±3.4 | 46.4±6.1 | 84.0±5.4 |

**[Table 18]**

| Sample concentration (*µ*g/ml) | | GRO-α production (%) | |
|---|---|---|---|
| | | Yellow dust | PM2.5 |
| Control | | 100.0±6.0 | 100.0±6.0 |
| Group treated with air pollutants | | 186.7±7.8 | 156.2±4.5 |
| *Citrus sunki* peel | 52.5 | 75.8±3.2 | 92.2±2.9 |
| | 105 | 62.6±4.1 | 64.9±4.3 |
| | 210 | 52.8±2.9 | 32.1±4.3 |
| *Sceptridium ternatum* (or *Botrychium ternatum*) | 52.5 | 90.2±1.7 | 98.2±3.2 |
| | 105 | 75.8±2.9 | 81.8±1.4 |
| | 210 | 68.7±2.2 | 65.6±3.5 |
| *Korthalsella japonica* | 52.5 | 53.3±1.3 | 74.3±2.7 |
| | 105 | 43.5±1.5 | 38.9±1.2 |
| | 210 | 39.6±2.6 | 27.2±2.6 |
| Mixture (1:1:1) | 52.5 | 58.1±5.6 | 55.7±2.9 |
| | 105 | 53.0±3.7 | 37.3±6.0 |
| | 210 | 45.5±1.1 | 20.0±1.6 |
| Mixture (1:1:2) | 52.5 | 68.9±2.2 | 66.8±5.2 |
| | 105 | 64.1±2.2 | 52.7±2.6 |
| | 210 | 55.6±1.4 | 28.8±1.9 |

**[Table 19]**

| Sample concentration (*µ*g/ml) | | GM-CSF production (%) | | | |
|---|---|---|---|---|---|
| | | Yellow dust | TSP | PM2.5 | TSP |
| Control | | 100.0±8.4 | 100.0±8.4 | 100.0±8.4 | 100.0±8.4 |
| Group treated with air pollutants | | 254.8±10.7 | 165.0±5.0 | 149.6±7.9 | 165.0±5.0 |
| *Citrus sunki* peel | 52.5 | 61.2±3.7 | 72.1±5.0 | 70.0±3.8 | 61.2±3.7 |
| | 105 | 54.4±0.7 | 64.6±1.3 | 63.2±1.4 | 54.4±0.7 |
| | 210 | 52.5±2.0 | 59.1±4.6 | 56.1±1.2 | 52.5±2.0 |
| *Sceptridium ternatum* (or *Botrychium ternatum*) | 52.5 | 146.4±3.4 | 147.6±3.3 | 127.2±2.2 | 146.4±3.4 |
| | 105 | 129.2±4.0 | 133.4±0.6 | 113.6±4.3 | 129.2±4.0 |
| | 210 | 118.6±7.1 | 113.7±4.1 | 105.8±1.6 | 118.6±7.1 |
| *Korthalsella japonica* | 52.5 | 175.8±3.3 | 202.5±2.6 | 167.2±2.5 | 175.8±3.3 |
| | 105 | 165.1±4.5 | 181.7±9.4 | 153.1±2.3 | 165.1±4.5 |
| | 210 | 149.0±3.2 | 148.2±6.5 | 132.4±2.5 | 149.0±3.2 |
| Mixture (1:1:1) | 52.5 | 68.8±2.8 | 69.6±2.5 | 81.5±3.3 | 68.8±2.8 |
| | 105 | 63.3±3.0 | 60.6±0.2 | 67.8±0.5 | 63.3±3.0 |
| | 210 | 60.5±2.0 | 55.3±3.4 | 62.5±1.2 | 60.5±2.0 |
| Mixture (1:1:2) | 52.5 | 64.3±1.0 | 64.6±2.0 | 72.2±2.2 | 64.3±1.0 |
| | 105 | 60.4±1.8 | 58.9±2.9 | 63.2±0.6 | 60.4±1.8 |
| | 210 | 53.7±0.7 | 53.4±3.2 | 58.0±0.8 | 53.7±0.7 |

### Experimental Example 5. Separation and identification of the active substances of Citrus sunki peel, Sceptridium ternatum (or Botrychium ternatum) and Korthalsella japonica extracts

### (1) Separation and identification of the active substances of ethanol extract of Citrus sunki peel

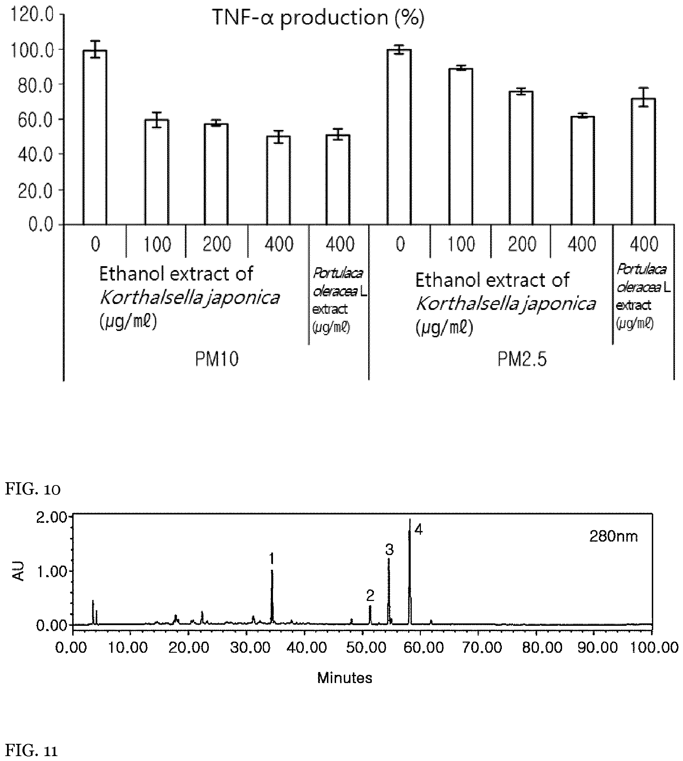

A chromatogram of the ethanol extract of *Citrus sunki* peel obtained through Example 1, which was analyzed with HPLC-UVD at 280nm under a concentration gradient condition of acetonitrile and distilled water by using a C18 column having a diameter of 4.6 mm and a length of 25 cm, was shown in FIG. 10, and it was identified that each peak was hesperidine (peak no. 1), tetramethyl-O-isoscutellarein (peak no. 2), nobiletin (peak no. 3) and tangeretin (peak no. 4) by analyzing spectroscopic data for four major peaks through HPLC-ESI-MS.

### (2) Separation and identification of the active substances of ethanol extract of Sceptridium ternatum (or Botrychium ternatum)

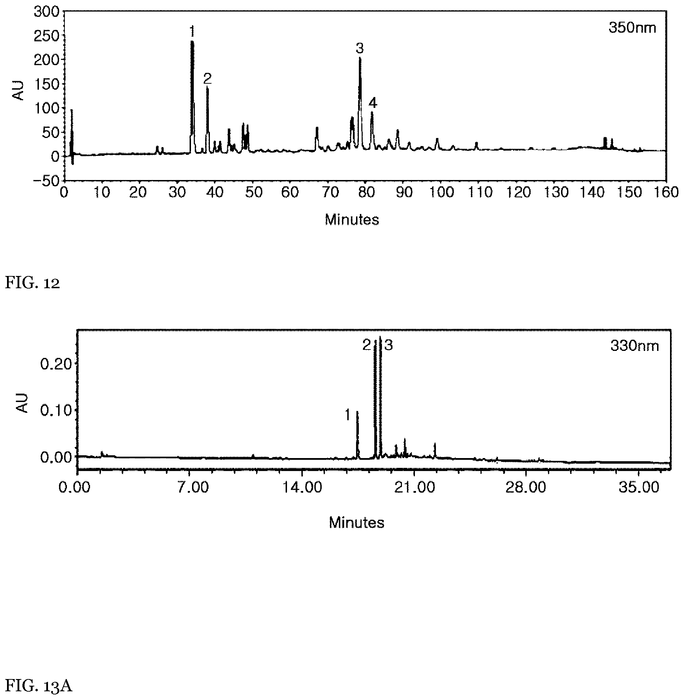

A chromatogram of the ethanol extract of *Sceptridium ternatum* (or *Botrychium ternatum*) obtained through Example 1, which was analyzed with HPLC-UVD at 350 nm under a concentration gradient condition of methanol and distilled water by using a C18 column having a diameter of 4.6 mm and a length of 10 cm, was shown in FIG. 11, and flavonoid glycosides were identified by analyzing spectroscopic data for four major peaks through HPLC-ESI-MS, wherein results thereof were shown in a following Table 20.

**[Table 20]**

| Peak No. | Compound | [M+H]⁺ | Theoretical mass | Observed mass | Mass difference |
|---|---|---|---|---|---|
| 1 | Quercetin-3-O-β-glucosyl(1→2)-α-rhamnoside-7-O-α-rhamnoside | C₃₃H₄₁O₂₀ | 757.2191 | 757.2241 | 5 mmu |
| 2 | Quercetin-3-O-β-glucosyl(1→2)-α-rhamnoside-7-O-β-glucoside | C₃₃H₄₁O₂₁ | 773.2140 | 773.2181 | 4 mmu |
| 3 | Ternatumoside XI | C₄₈H₅₇O₂₇ | 1065.3087 | 1065.3270 | 18 mmu |
| 4 | Ternatumoside VI or Ternatumoside XII | C₅₇H₆₃O₂₉ | 1211.3455 | 1211.3664 | 20 mmu |

### (3) Separation and identification of the active substances of ethanol extract of Korthalsella japonica

A chromatogram of the ethanol extract of *Korthalsella japonica* obtained through Example 1, which was analyzed with HPLC-UVD at 330 nm under a concentration gradient condition of acetonitrile and distilled water by using a C18 column having a diameter of 3.9 mm and a length of 30 cm, was shown in FIG. 12, and three flavonoid glycosides of Lucenin-2 (peak no. 1), Vicenin-2 (peak no. 2) and Stellarin-2 (peak no. 3) were identified and separated by comparing and analyzing spectroscopic data through HPLC-ESI-MS, MS/MS and existing documents (Ferreres et al., 2003, Parejo et al., 2004) for three major peaks, wherein a chemical structural formula thereof was shown in a following Formula 1.

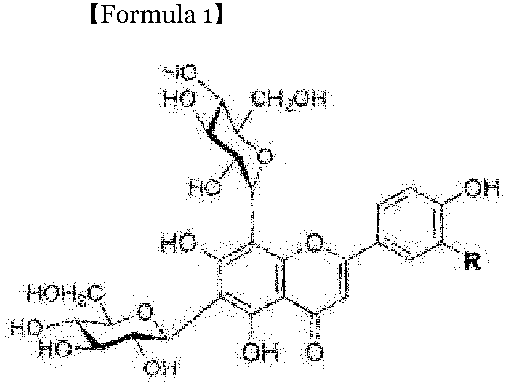

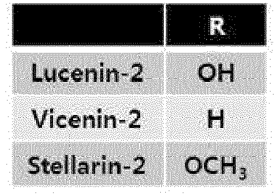

### Experimental Example 6. Evaluation of the ability of active substances separated from the ethanol extract of Korthalsella japonica to alleviate inflammation caused by yellow dust or fine particulate

A following experiment was performed to identify if three types of flavonoid glycoside separated from the ethanol extract of *Korthalsella japonica* are major ingredients exhibiting an anti-inflammatory effect.

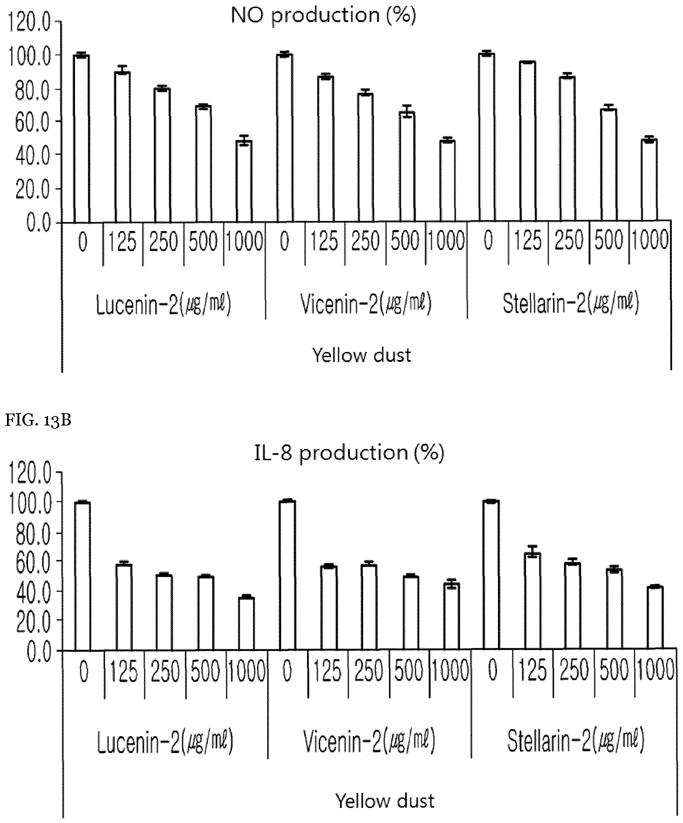

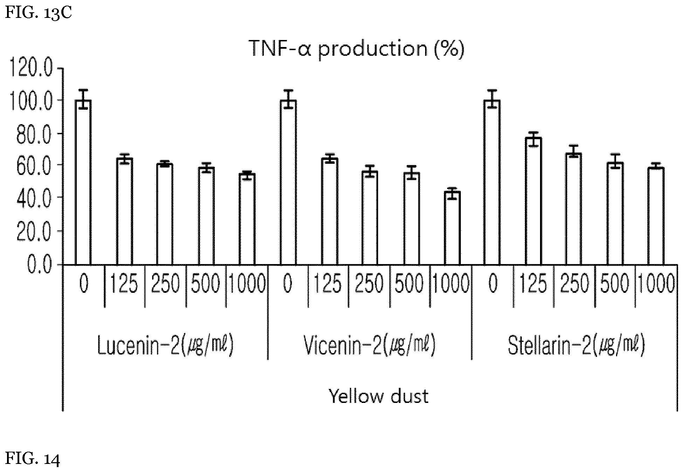

The Raw 264.7 cells and HaCaT cells were cultured for 24 hours, after which resulting cells were treated with each substance at a concentration of 125-1000*µ*g/ml, in which its cytotoxicity does not appear. Then, resulting cells were treated with 100x-diluted distilled water extract of yellow dust and 100x-diluted chloroform extract of yellow dust, after which resulting treated cells were cultured again for 24 hours. As shown in Experimental Example 3, the inhibitory effect on the production of NO, IL-8 and TNF-α was measured, wherein results thereof were shown in FIG. 13.

As shown in FIG. 13, three types of flavonoid glycoside separated from the ethanol extract of *Korthalsella japonica* exhibited an inhibitory effect on the production of NO, IL-8 and TNF-α in a concentration-dependent way. Based on such results, it was identified that the three types of flavonoid glycoside separated from the ethanol extract of *Korthalsella japonica* could effectively inhibit the production of NO, IL-8 and TNF-α.

### Experimental Example 7. Identification of the production of other inflammatory cytokines by means of yellow dust and fine particulate

A cytokine array experiment was performed in order to see which cytokines were further produced in addition to the inflammatory cytokines produced by yellow dust and fine particulate identified in the above Experimental Example 1 and how they varied depending on types of yellow dust and fine particulate. The HaCaT cells were seeded at 2.0 x 10⁵ cells/well into a 96-well plate, after which resulting cells were cultured under a condition of 37°C and 5% CO□ for 24 hours. The resulting cells were treated respectively with 100x-diluted distilled water extracts of yellow dust, TSP and PM2.5, after which resulting treated cells were further cultured for 24 hours, such that the resulting culture media thereof were collected to evaluate their production amounts of G-CSF (Granulocyte Colony Stimulating Factor), GM-CSF (Granulocyte-Macrophage Colony Stimulating Factor), GRO (Growth-Regulated Protein), GRO-α (Growth-Regulated Protein-α), IL-1α (Interleukin-1α), IL-2 (Interleukin-2), IL-3 (Interleukin-3), IL-5 (Interleukin-5), IL-6 (Interleukin-6), IL-7 (Interleukin-7), IL-8 (Interleukin-8), IL-10 (Interleukin-10), IL-13 (Interleukin-13), IL-15 (Interleukin-15), INF-γ (Interferon-y), MCP-1 (Monocyte Chemoattractant Protein-1), MCP-2 (Monocyte Chemoattractant Protein-2), MCP-3 (Monocyte Chemoattractant Protein-3), CXCL9 (Chemokine Ligand 9; MIG), CCL5 (Chemokine Ligand 5; RANTES), TGF-β1 (Transforming Growth Factor-β1), TNF-α (Tumor Necrosis Factor-a) and TNF-β (Tumor Necrosis Factor-β) by using a Human Cytokine Antibody Array Kit (Raybiotech).

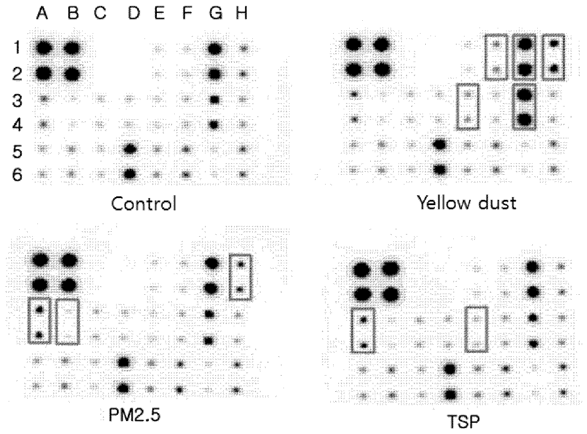

Results of production rate of each cytokine compared to a control untreated with the distilled water extracts of yellow dust, TSP and PM2.5 were shown in Table 21 and FIG. 14. A positive control was treated with biotinylated antibody to identify if an experiment was well performed, a negative control was treated only with buffer to see its response baseline, and a blank was not treated at all to see background responses.

**[Table 21]**

| | A | B | C | D | E | F | G | H |
|---|---|---|---|---|---|---|---|---|
| 1 | Positive control | Positive control | Negative control | Negative control | G-CSF | GM-CSF | GRO | GRO-α |
| 2 | | | | | Yellow dust:120.2 | Yellow dust:290.8 | Yellow dust:132.5 | Yellow dust:255.7 |
| | | | | | TSP:116.9 | TSP:117.1 | TSP:95.2 | TSP:76.3 |
| | | | | | PM2.5:122.8 | PM2.5:120. 2 | PM2.5:115. 0 | PM2.5:135. 9 |
| 3 | IL-1α | IL-2 | IL-3 | IL-5 | IL-6 | IL-7 | IL-8 | IL-10 |
| 4 | Yellow dust:119.0 | Yellow dust:118.7 | Yellow dust:113.8 | Yellow dust:102.3 | Yellow dust:263.9 | Yellow dust:94.8 | Yellow dust:437.7 | Yellow dust:94.6 |
| | TSP:121.1 | TSP:147.3 | TSP:123.7 | TSP:120.0 | TSP:1.35.3 | TSP:111.6 | TSP:91.2 | TSP:111.8 |
| | PM2.5:145. 7 | PM2.5:132. 7 | PM2.5:124. 0 | PM2.5:115. 2 | PM2.5:116. 4 | PM2.5:108. 7 | PM2.5:120. 9 | PM2.5:102. 5 |
| 5 | IL-13 | IL-15 | IFN-γ | MCP-1 | MCP-2 | MCP-3 | CXCL9 | CCL5 |
| 6 | Yellow dust:108.1 | Yellow dust: 109.8 | Yellow dust:96.5 | Yellow dust:127.0 | Yellow dust: 101.3 | Yellow dust:93.0 | Yellow dust:97.8 | Yellow dust:100.4 |
| | TSP:112.7 | TSP:121.7 | TSP:106.8 | TSP:113.0 | TSP:115.8 | TSP:101.8 | TSP:98.4 | TSP:104.1 |
| | PM2.5:112. 4 | PM2.5:126. 3 | PM2.5:100. 3 | PM2.5:105. 8 | PM2.5:123. 3 | PM2.5:110. 4 | PM2.5:112. 7 | PM2.5:111. 3 |
| 7 | TGF β 1 | TNF α | TNF β | Blank | Blank | Blank | Blank | Positive control |
| 8 | Yellow dust:105.3 | Yellow dust:108.4 | Yellow dust:97.6 | | | | | |
| | TSP:113.2 | TSP:104.1 | TSP:107.0 | | | | | |
| | PM2.5:101. 1 | PM2.5:126. 1 | PM2.5:111. 3 | | | | | |

| | | | | | | | | |
|---|---|---|---|---|---|---|---|---|
| * The figures of the above Table indicate a production rate of each cytokine compared to the control 100. | | | | | | | | |

As shown in Table 21 and FIG. 14, it was shown that the yellow dust extract increased a production amount of G-CSF, GM-CSF, GRO, GRO-α, IL-1α, IL-2, IL-6, IL-8 and MCP-1, the TSP extract increased a production amount of G-CSF, GM-CSF, IL-1α, IL-2, IL-3, IL-5, IL-6, IL-15 and MCP-2, the PM2.5 extract increased a production amount of G-CSF, GM-CSF, GRO, GRO-α, IL-1α, IL-2, IL-3, IL-5, IL-6, IL-8, IL-15, MCP-2 and TNF-α compared to a production amount of the control.

Accordingly, it was identified that the production of different cytokines occurred depending on types of yellow dust and fine particulate. This means that inflammatory responses with different properties occurred depending on stimulus-causing substances, and that the inflammatory responses caused by yellow dust and fine particulate were distinctively different from general inflammatory responses.

### Preparing Example: Cosmetic composition comprising ethanol extracts of Citrus sunki peel, Sceptridium ternatum (or Botrychium ternatum) and Korthalsella japonica

On the grounds of the above Experimental Examples, various formulations of cosmetic composition were prepared, comprising the ethanol extracts of *Citrus sunki* peel, *Sceptridium ternatum* (or *Botrychium ternatum*) and *Korthalsella japonica* obtained from Example 1 as described in following Tables 22 to 33. Following Formulation Examples are set forth just as an example for the more specific description of the present invention, but are not to be construed to limit the content of the present invention

As seen in the above Experimental Examples, the ethanol extracts of *Citrus sunki* peel, *Sceptridium ternatum* (or *Botrychium ternatum*) and *Korthalsella japonica* showed a difference in relative effects in inhibiting the production of NO and the production of inflammatory cytokines according to each of air pollutants (yellow dust, TSP, PM10 and PM2.5), which are causes of inflammation. Thus, it is possible to prepare a cosmetic composition comprising a mixture that combines all the ethanol extracts of *Citrus sunki* peel, *Sceptridium ternatum* (or *Botrychium ternatum)* and *Korthalsella japonica.*

### Formulation Example 1. High viscosity emulsion formulation

A high viscosity emulsion formulation of a mixture containing each or all of the ethanol extracts of *Citrus sunki* peel, *Sceptridium ternatum* (or *Botrychium ternatum*) and *Korthalsella japonica,* obtained from Example 1, was prepared by means of a conventional method according to composition ingredients and ratios shown in following Tables 22 to 25.

### < Method for preparing a high viscosity emulsion formulation >

① Water and oil phases were respectively heated to be uniformly mixed and dissolved.
② The oil phase was inserted into the water phase at 75°C to be mixed and solubilized together.
③ An additive phase I, in which a mixture containing each or all of the ethanol extracts of *Citrus sunki* peel, *Sceptridium ternatum* (or *Botrychium ternatum*) and *Korthalsella japonica* was dissolved, was inserted into the resulting mixed phase of ② at 50°C and mixed together, after which an additive phase II was mixed therein together.

**[Table 22]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Oil phase** | PEG-100 stearate | 0.1 - 2 |
| | Glyceryl stearate | 0.1 - 2 |
| | Polysorbate 60 | 0.1 - 2 |
| | Stearic acid | 0.1 - 2 |
| | Cetearyl alcohol | 0.1 - 2 |
| | Caprylic/Capric Triglyceride | 10 - 30 |
| | Tocopheryl acetate | 0.1 - 0.5 |
| **Additive phase I** | Ethanol extract of *Citrus sunki* peel | 0.25 |
| **Additive phase II** | Fragrance | Suitable amount |
| | Preservative | Suitable amount |
| | Other additive | Suitable amount |

**[Table 23]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Oil phase** | PEG-100 stearate | 0.1 - 2 |
| | Glyceryl stearate | 0.1 - 2 |
| | Polysorbate 60 | 0.1 - 2 |
| | Stearic acid | 0.1 - 2 |
| | Cetearyl alcohol | 0.1 - 2 |
| | Caprylic/Capric Triglyceride | 10 - 30 |
| | Tocopheryl acetate | 0.1 - 0.5 |
| **Additive phase I** | Ethanol extract of *Sceptridium ternatum* (or *Botrychium ternatum*) | 0.25 |
| **Additive phase II** | Fragrance | Suitable amount |
| | Preservative | Suitable amount |
| | Other additive | Suitable amount |

**[Table 24]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Oil phase** | PEG-100 stearate | 0.1 - 2 |
| | Glyceryl stearate | 0.1 - 2 |
| | Polysorbate 60 | 0.1 - 2 |
| | Stearic acid | 0.1 - 2 |
| | Cetearyl alcohol | 0.1 - 2 |
| | Caprylic/Capric Triglyceride | 10 - 30 |
| | Tocopheryl acetate | 0.1 - 0.5 |
| **Additive phase I** | Ethanol extract of *Korthalsella japonica* | 0.25 |
| **Additive phase II** | Fragrance | Suitable amount |
| | Preservative | Suitable amount |
| | Other additive | Suitable amount |

**[Table 25]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Oil phase** | PEG-100 stearate | 0.1 - 2 |
| | Glyceryl stearate | 0.1 - 2 |
| | Polysorbate 60 | 0.1 - 2 |
| | Stearic acid | 0.1 - 2 |
| | Cetearyl alcohol | 0.1 - 2 |
| | Caprylic/Capric Triglyceride | 10 - 30 |
| | Tocopheryl acetate | 0.1 - 0.5 |
| **Additive phase I** | Ethanol extract of *Citrus sunki* peel | 0.25 |
| | Ethanol extract of *Sceptridium ternatum* (or *Botrychium ternatum*) | |
| | Ethanol extract of *Korthalsella japonica* | |
| **Additive phase II** | Fragrance | Suitable amount |
| | Preservative | Suitable amount |
| | Other additive | Suitable amount |

### Formulation Example 2. Low viscosity emulsion formulation

A low viscosity emulsion formulation of a mixture containing each or all of the ethanol extracts of *Citrus sunki* peel, *Sceptridium ternatum* (or *Botrychium ternatum*) and *Korthalsella japonica,* obtained from Example 1, was prepared by means of a conventional method according to composition ingredients and ratios shown in following Tables 26 to 29.

### < Method for preparing a low viscosity emulsion formulation >

① Water and oil phases were respectively heated to be uniformly mixed and dissolved.
② The oil phase was inserted into the water phase at 75°C to be mixed and solubilized together.
③ An additive phase I, in which a mixture containing each or all of the ethanol extracts of *Citrus sunki* peel, *Sceptridium ternatum* (or *Botrychium ternatum*) and *Korthalsella japonica* was dissolved, was inserted into the resulting mixed phase of ② at 50°C and mixed together, after which an additive phase II was mixed therein together.

**[Table 26]**

| **Phase** | **Ingredient** | **Content (%)** |
|---|---|---|
| **Water phase** | Purified water | To 100 |
| | Ceteareth-6 olivate | 0.1 - 3 |
| | Moisturizing ingredient | 10 - 25 |
| | Thickener | Suitable amount |
| | Chelating agent | Suitable amount |
| **Oil phase** | PEG-100 stearate | 0.1 - 1 |
| | Glyceryl stearate | 0.1 - 1 |
| | Polysorbate 60 | 0.1 - 1 |
| | Cetyl alcohol | 0.1 - 1 |
| | Behenyl alcohol | 0.1 - 1 |
| | Squalane | 5-20 |
| | Tocopheryl acetate | 0.1 - 0.5 |
| **Additive phase I** | Ethanol extract of *Citrus sunki* peel | 0.25 |
| **Additive phase II** | Fragrance | Suitable amount |
| | Preservative | Suitable amount |
| | Other additive | Suitable amount |

**[Table 27]**

| **Phase** | **Ingredient** | **Content (%)** |
|---|---|---|
| **Water phase** | Purified water | To 100 |
| | Ceteareth-6 olivate | 0.1 - 3 |
| | Moisturizing ingredient | 10 - 25 |
| | Thickener | Suitable amount |
| | Chelating agent | Suitable amount |
| **Oil phase** | PEG-100 stearate | 0.1 - 1 |
| | Glyceryl stearate | 0.1 - 1 |
| | Polysorbate 60 | 0.1 - 1 |
| | Cetyl alcohol | 0.1 - 1 |
| | Behenyl alcohol | 0.1 - 1 |
| | Squalane | 5 - 20 |
| | Tocopheryl acetate | 0.1 - 0.5 |
| **Additive phase I** | Ethanol extract of *Sceptridium ternatum* (or *Botrychium ternatum*) | 0.25 |
| **Additive phase II** | Fragrance | Suitable amount |
| | Preservative | Suitable amount |
| | Other additive | Suitable amount |

**[Table 28]**

| **Phase** | **Ingredient** | **Content (%)** |
|---|---|---|
| **Water phase** | Purified water | To 100 |
| | Ceteareth-6 olivate | 0.1 - 3 |
| | Moisturizing ingredient | 10 - 25 |
| | Thickener | Suitable amount |
| | Chelating agent | Suitable amount |
| **Oil phase** | PEG-100 stearate | 0.1 - 1 |
| | Glyceryl stearate | 0.1 - 1 |
| | Polysorbate 60 | 0.1 - 1 |
| | Cetyl alcohol | 0.1 - 1 |
| | Behenyl alcohol | 0.1 - 1 |
| | Squalane | 5 - 20 |
| | Tocopheryl acetate | 0.1 - 0.5 |
| **Additive phase I** | Ethanol extract of *Korthalsella japonica* | 0.25 |
| **Additive phase II** | Fragrance | Suitable amount |
| | Preservative | Suitable amount |
| | Other additive | Suitable amount |

**[Table 29]**

| **Phase** | **Ingredient** | **Content (%)** |
|---|---|---|
| **Water phase** | Purified water | To 100 |
| | Ceteareth-6 olivate | 0.1 - 3 |
| | Moisturizing ingredient | 10 - 25 |
| | Thickener | Suitable amount |
| | Chelating agent | Suitable amount |
| **Oil phase** | PEG-100 stearate | 0.1 - 1 |
| | Glyceryl stearate | 0.1 - 1 |
| | Polysorbate 60 | 0.1 - 1 |
| | Cetyl alcohol | 0.1 - 1 |
| | Behenyl alcohol | 0.1 - 1 |
| | Squalane | 5 - 20 |
| | Tocopheryl acetate | 0.1 - 0.5 |
| **Additive phase I** | Ethanol extract of *Citrus sunki* peel | 0.25 |
| | Ethanol extract of *Sceptridium ternatum* (or *Botrychium ternatum*) | |
| | Ethanol extract of *Korthalsella japonica* | |
| **Additive phase II** | Fragrance | Suitable amount |
| | Preservative | Suitable amount |
| | Other additive | Suitable amount |

### Formulation Example 3. Solubilization formulation

A solubilization formulation of a mixture containing each or all of the ethanol extracts of *Citrus sunki* peel, *Sceptridium ternatum* (or *Botrychium ternatum*) and *Korthalsella japonica,* obtained from Example 1, was prepared by means of a conventional method according to composition ingredients and ratios shown in following Tables 30 to 33.

### < Method for preparing a solubilization formulation >

① Water and solubilization phases were respectively and uniformly mixed and dissolved.
② The solubilization phase was inserted into the water phase and mixed together to obtain an oil-in-water type emulsion.
③ An additive phase I, in which a mixture containing each or all of the ethanol extracts of *Citrus sunki* peel, *Sceptridium ternatum* (or *Botrychium ternatum*) and *Korthalsella japonica* was dissolved, was solubilized, after which the resulting additive phase I was inserted into the resulting mixed phase of ② above and mixed together, such that an additive phase II was mixed therein together and completed.

**[Table 30]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Solubilizatio n phase** | PEG-60 hydrogenated castor oil | 0 - 2 |
| | PEG-40 hydrogenated castor oil | 0 - 2 |
| | Polyhydric alcohol | 0 - 10 |
| | Fragrance | Suitable amount |
| | Ethanol | 0 - 20 |
| **Additive phase I** | Ethanol extract of *Citrus sunki* peel | 0.25 |
| **Additive phase II** | Preservative | Suitable amount |
| | Other additive | Suitable amount |

**[Table 31]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Solubilizatio n phase** | PEG-60 hydrogenated castor oil | 0 - 2 |
| | PEG-40 hydrogenated castor oil | 0 - 2 |
| | Polyhydric alcohol | 0 - 10 |
| | Fragrance | Suitable amount |
| | Ethanol | 0 - 20 |
| **Additive phase I** | Ethanol extract of *Sceptridium ternatum* (or *Botrychium ternatum*) | 0.25 |
| **Additive phase II** | Preservative | Suitable amount |
| | Other additive | Suitable amount |

**[Table 32]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Solubilizatio n phase** | PEG-60 hydrogenated castor oil | 0 - 2 |
| | PEG-40 hydrogenated castor oil | 0 - 2 |
| | Polyhydric alcohol | 0 - 10 |
| | Fragrance | Suitable amount |
| | Ethanol | 0 - 20 |
| **Additive phase I** | Ethanol extract of *Korthalsella japonica* | 0.25 |
| **Additive phase II** | Preservative | Suitable amount |
| | Other additive | Suitable amount |

**[Table 33]**

| **Phase** | **Ingredient** | **Content (%)** |
|---|---|---|
| **Water phase** | Purified water | To 100 |
| | Moisturizing ingredient | 10 - 25 |
| | Thickener | Suitable amount |
| | Chelating agent | Suitable amount |
| **Solubilizatio n phase** | PEG-60 hydrogenated castor oil | 0 - 2 |
| | PEG-40 hydrogenated castor oil | 0 - 2 |
| | Polyhydric alcohol | 0 - 10 |
| | Fragrance | Suitable amount |
| | Ethanol | 0 - 20 |
| **Additive phase I** | Ethanol extract of *Citrus sunki* peel | 0.25 |
| | Ethanol extract of *Sceptridium ternatum* (or *Botrychium ternatum*) | |
| | Ethanol extract of *Korthalsella japonica* | |
| **Additive phase II** | Preservative | Suitable amount |
| | Other additive | Suitable amount |

### Experimental Example 7. Formulation stability of cosmetic composition

Cosmetic compositions of the above Formulation Examples 1, 2 and 3 were stored for 12 weeks under a condition of 4°C, 30°C, 45°C and sunlight, and changes in the color, odor and property of a formulation were observed through visual and sensory evaluations, wherein results thereof were indicated in Tables 34 to 36.

As shown in Tables 34 to 36, it was identified that cosmetic compositions with the high viscosity emulsion formulation, the low viscosity emulsion formulation and the solubilization formulation of the mixture containing each or all of the ethanol extracts of *Citrus sunki* peel, *Sceptridium ternatum* (or *Botrychium ternatum*) and *Korthalsella japonica* had an excellent stability without any change in the color, odor and property, even if they were stored for a long period of time under high temperature and sunlight conditions.

### <Evaluation criteria for stability>

No change: ○
Slight change: Δ
Significant change: X

**[Table 34]**

| Elapsed time (weeks) | Extract | High viscosity emulsion formulation | | | | | | | | | | | |
|---|---|---|---|---|---|---|---|---|---|---|---|---|---|
| | | Change in odor | | | | Change in color | | | | Change in formulation | | | |
| | | 4°C | 30°C | 45°C | Sunlig ht | 4°C | 30°C | 45°C | Sunlig ht | 4°C | 30°C | 45°C | Sunlig ht |
| 1 | *Citrus sunki* peel | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Sceptridiu m ternatum* (or *Botrychium ternatum*) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Korthalsell a japonica* | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | Mixture (1:1:1) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |

**[Table 35]**

| Elapsed time (weeks) | Extract | Low viscosity emulsion formulation | | | | | | | | | | | |
|---|---|---|---|---|---|---|---|---|---|---|---|---|---|
| | | Change in odor | | | | Change in color | | | | Change in formulation | | | |
| | | 4°C | 30°C | 45°C | Sunlig ht | 4°C | 30°C | 45°C | Sunlig ht | 4°C | 30°C | 45°C | Sunlig ht |
| 1 | *Citrus sunki* peel | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Sceptridiu m ternatum* (or *Botrychium ternatum*) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Korthalsell a japonica* | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | Mixture (1:1:1) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |

**[Table 36]**

| Elapsed time (weeks) | Extract | Solubilization formulation | | | | | | | | | | | |
|---|---|---|---|---|---|---|---|---|---|---|---|---|---|
| | | Change in odor | | | | Change in color | | | | Change in formulation | | | |
| | | 4°C | 30°C | 45°C | Sunlig ht | 4°C | 30°C | 45°C | Sunlig ht | 4°C | 30°C | 45°C | Sunlig ht |
| 1 | *Citrus sunki* peel | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Sceptridiu m ternatum* (or *Botrychium ternatum*) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | *Korthalsell a japonica* | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |
| 1 | Mixture (1:1:1) | ○ | ○ | ○ | ○ | ○ | ○ | ○ | ○ | ○ | ○ | ○ | ○ |
| 2 | | ○ | ○ | ○ | ○ | ○ | ○ | ○ | ○ | ○ | ○ | ○ | ○ |
| 4 | | ○ | ○ | ○ | ○ | ○ | ○ | ○ | ○ | ○ | ○ | ○ | ○ |
| 8 | | ○ | ○ | ○ | ○ | ○ | ○ | ○ | ○ | ○ | ○ | ○ | ○ |
| 12 | | ○ | ○ | ○ | ○ | ○ | ○ | ○ | ○ | ○ | ○ | ○ | ○ |

### Experimental Example 8. Clinical evaluation of the effectiveness of cosmetic composition comprising ethanol extracts of Citrus sunki peel, Sceptridium ternatum (or Botrychium ternatum) and Korthalsella japonica to alleviate stimulus after causing skin inflammation by means of yellow dust or fine particulate

To evaluate effectiveness of a cosmetic composition comprising ethanol extracts of *Citrus sunki* peel, *Sceptridium ternatum* (or *Botrychium ternatum*) and *Korthalsella japonica,* a clinical evaluation was performed after being divided into two parts: first and second evaluations. The first test was performed with a total of 20 subjects (11 males and 9 females), while the second test was performed with a total of 22 subjects (9 males and 13 females).

In the first test, to identify if skin inflammatory responses are caused from yellow dust or fine particulate, a sodium lauryl sulfate (SLS) solution and an SLS + fine particulate solution were applied to different regions respectively to measure a change in a degree of skin redness (a*), which is a factor to identify whether the skin inflammatory responses occur or not, before application, in 1 day after application and in 2 days after application.

In the second test, to identify if a composition comprising natural plant extracts of the present invention is effective in skin inflammation caused from yellow dust or fine particulate, an SLS + fine particulate solution and an SLS + fine particulate solution + test product were applied to different regions respectively to measure a degree of skin redness (a*), an amount of skin erythema (E.I) or an amount of transepidermal water loss (TEWL), which are factors to identify whether the skin inflammatory responses occur or not, in 1 day after use, 2 days after use and 7 days after use.

In the first and second tests, the SLS was a product at a concentration of 98% prepared by Wako Inc., Japan, the fine particulate solution was prepared from Example 2, in which the yellow dust, TSP, PM2.5 and PM10 extracts were 100x to 500x diluted. The test product was a cosmetic composition comprising ethanol extracts of *Citrus sunki* peel, *Sceptridium ternatum* (or *Botrychium ternatum)* and *Korthalsella japonica* in Table 25 of the above Formulation Example 1. In the above first test, the application was performed on the left musculus biceps region of a subject, while the application of the second test was performed on the right musculus biceps region of the subject.

In case of the first experiment, a total of four visits were made. In the first visit, a measurement before product use and an attachment of skin damage patch were performed. In the second visit, a removal of the patch, a measurement of skin right after caused damage and an application of fine particulate solution were performed. In the third visit, a measurement in one day after the application of fine particulate solution was performed. In the fourth visit, a measurement in two days after the application of the fine particulate solution as well as an identification of abnormal responses were performed.

In case of the second experiment, a total of five visits were made. In the first visit, a measurement before product use and an attachment of skin damage patch were performed. In the second visit, a removal of the patch, a measurement of skin right after caused damage, an application of fine particulate solution and an application of the product were performed. In the third visit, a measurement in one day after the application of fine particulate solution was performed. In the fourth visit, a measurement in two days after the application of the fine particulate solution was performed. In the fifth visit, a measurement in seven days after the application of the fine particulate solution as well as an identification of abnormal responses were performed.

### Experimental Example 8-1. Identification of skin inflammatory responses caused from fine particulate through application of fine particulate solution (first test)

### (1) Identification of changes in a degree of skin redness (a*)

To identify changes in a degree of skin redness by means of an application of SLS and an application of SLS + fine particulate solution, a skin color was measured before the application, right after the application, in one day after the application and in two days after the application by using Chroma Meter CM700d (Konica Minolta Sensing Americas, Inc, USA). At every measurement, a degree of skin redness on the same region was measured three times, such that an average thereof was used as evaluation data. The degree of skin redness was indicated by measuring a* value, which indicates a degree of redness in a CIE L*a*b* color space based on human color perception. If the a* value increases, it means that a corresponding color is visually more red. Results of the degree of skin redness measured for 20 subjects were indicated as average ± standard error (SE), which were shown in a following Table 37.

**[Table 37]**

| Classification | Application of SLS | | Application of SLS + fine particulate solution | | Significance probability between application of SLS and application of SLS + fine particulate solution |
|---|---|---|---|---|---|
| | Degree of skin redness (a*) | Significance Probability | Degree of skin redness (a*) | Significance Probability | |
| Test of within-subject effect | - | 0.022# | - | 0.000* | |
| Before application | 6.357±1.231 | - | 6.714±0.972 | - | |
| Right after application | 9.578±1.891 | - | 10.175±1.923 | - | 0.329 |
| 1 day after application | 10.423+1.981 | 0.008# | 12.099±2.010 | 0.000* | 0.011+ |
| 2 days after application | 10.753±1.953 | 0.033 | 12.158±1.974 | 0.001* | 0.029+ |

| | | | | | |
|---|---|---|---|---|---|
| *: p<0.05 Post-hoc test results of a non-parametric test, which was repeatedly measured by means of a parametric method ANOVA according to a test of normality #: p<0.025 Results of conducting a Friedman test as a non-parametric method and then a post-hoc test as a non-parametric test method according to a test of normality +: p<0.05 Independent sample t test result | | | | | |

As shown in Table 37, a measured value for the application of SLS and the application of SLS + fine particulate solution was significantly increased (p<0.025, p<0.05) in one day after the application, and the measured value for the application of SLS +fine particulate solution was significantly increased (p<0.05) even in two days after the application. It was identified that the measured value for the application of SLS + fine particulate solution was significantly higher (p<0.05) than the measured value for the application of SLS in one day and two days after the application.

Thus, it was identified that the degree of skin redness was significantly increased more in the application of the additional fine particulate solution compared to the application of SLS.

Based on the above results, it was identified that the fine particulate solution comprising yellow dust or fine particulate (TSP, PM2.5 and PM10) significantly increased the degree of skin redness, which is an inflammatory response factor.

### Experimental Example 8-2. Identification of the effect of composition comprising natural plant extracts of the present invention on skin inflammation caused from fine particulate (second test)

As seen from the changes in the degree of skin redness in the above Experimental Example 8-1, it was identified that a significant inflammatory response occurred to skin by means of the fine particulate solution, and a clinical test was performed to see if the natural plant extracts of the present invention were effective in skin inflammation caused from fine particulate.

### (1) Identification of changes in degree of skin redness (a*)

To identify changes in the degree of skin redness by means of an application of SLS + fine particulate solution and an application of SLS + fine particulate solution + test product, a skin color was measured before the application, right after the application, in one day after the application, in two days after the application and in seven days after the application by using Chroma Meter CM700d, which is a skin color measuring instrument. At every measurement, the degree of skin redness on the same region was measured three times. Results of the degree of skin redness measured for 22 subjects were indicated as average ± standard error (SE), which were shown in a following Table 38.

**[Table 38]**

| Classification | Application of SLS + fine particulate solution | | Application of SLS + fine particulate solution + test product | | Significance probability between application of SLS + fine particulate solution and application of SLS + fine particulate solution + test product |
|---|---|---|---|---|---|
| | Degree of skin redness (a*) | Significance Probability | Degree of skin redness (a*) | Significance Probability | |
| Test of within-subject effect | - | 0.001* | - | 0.000* | |
| Before application | 6.473±1.121 | - | 6.516±1.132 | - | |
| Right after application | 10.639±1.947 | - | 10.586±1.928 | - | 0.928 |
| 1 day after application | 12.073±1.920 | 0.000* | 11.886±2.033 | 0.000* | 0.756 |
| 2 days after application | 11.470±2.204 | 0.094 | 10.556±1.961 | 1 | 0.154 |
| 7 days after application | 10.845±2.193 | 1 | 8.870±2.033 | 0.000* | 0.003+ |

| | | | | | |
|---|---|---|---|---|---|
| *: p<0.05 Post-hoc test results of a non-parametric test, which was repeatedly measured by means of a parametric method ANOVA according to a test of normality #: p<0.025 Results of conducting a Friedman test as a non-parametric method and then a post-hoc test as a non-parametric test method according to a test of normality +: p<0.05 Independent sample t test result | | | | | |

As shown in Table 38, a measured value for the application of SLS + fine particulate solution and the application of SLS + fine particulate solution + test product was significantly increased (p<0.05) in one day after the application, and the measured value for the application of SLS + fine particulate solution + test product was significantly decreased (p<0.05) in seven days after use. It was identified that the measured value for the application of SLS + fine particulate solution + test product was significantly decreased (p<0.05) compared to the measured value for the application of SLS + fine particulate solution in seven days after the application.

Thus, it was identified that the application of the additional test product significantly decreased the degree of skin redness, and it was identified that the application of SLS + fine particulate solution + test product decreased the degree about 20.0% in seven days after the application compared to the application of SLS + fine particulate solution.

### (2) Identification of changes in skin erythema (E.I)

To identify changes in an amount of skin erythema by means of application of SLS + fine particulate solution and application of SLS + fine particulate solution + test product, an amount of the skin erythema was measured before application, right after application, in one day after application, in two days after application and in seven days after application in such a way that an amount of hemoglobin under the skin was measured with light reflected from a probe at 568 nm (green), 660 nm (red) and 880 nm (near-infrared) by using a skin color measuring instrument (narrow-band reflectance spectrophotometer) called Mexameter (Courage+Khazaka Electronic GmbH, Germany), which uses only a narrow band wavelength. At every measurement, the degree of skin erythema (E.I) on the same region was measured three times, such that an average thereof was used as evaluation data. Results of the degree of skin erythema measured for 22 subjects were indicated as average ± standard error (SE), which were shown in a following Table 39.

**[Table 39]**

| Classification | Application of SLS + fine particulate solution | | Application of SLS + fine particulate solution + test product | | Significance probability between application of SLS + fine particulate solution and application of SLS + fine particulate solution SLS+ test product |
|---|---|---|---|---|---|
| | Skin erythema (E.I) | Significance Probability | Skin erythema (E.I) | Significance Probability | |
| Test of within-subject effect | - | 0.000* | - | 0.000* | |
| Before application | 155.230±32.531 | - | 159.894±49.908 | - | |
| Right after application | 230.320±50.246 | - | 231.682±50.240 | - | 0.938 |
| 1 day after application | 301.000±61.231 | 0.000* | 291.720±67.237 | 0.000* | 0.636 |
| 2 days after application | 303.682±66.220 | 0.000* | 287.545±68.626 | 0.000* | 0.432 |
| 7 days after application | 303.182±83.330 | 0.000* | 251.652±78.974 | 0.534 | 0.040+ |

| | | | | | |
|---|---|---|---|---|---|
| *: p<0.05 Post-hoc test results of a non-parametric test, which was repeatedly measured by means of a parametric method ANOVA according to a test of normality #: p<0.025 Results of conducting a Friedman test as a non-parametric method and then a post-hoc test as a non-parametric test method according to a test of normality +: p<0.05 Independent sample t test result | | | | | |

As shown in Table 39, a measured value for the application of SLS + fine particulate and the application of SLS + fine particulate + test product was significantly increased (p<0.05) in one day and two days after the application, and the measured value for the application of SLS + fine particulate was significantly increased (p<0.05) in seven days after use. It was identified that the measured value for the application of SLS + fine particulate solution + test product was significantly decreased (p<0.05) compared to the measured value for the application of SLS + fine particulate in seven days after the application of fine particulate.

Thus, it was identified that the application of the additional test product significantly decreased a degree of skin erythema (E.I) and the application of SLS + fine particulate solution + test product decreased the degree about 16.8% in seven days after the application compared to the application of SLS + fine particulate solution.

### (3) Identification of changes in transepidermal water loss (TEWL)

To identify changes in a transepidermal water loss (TEWL) by means of application of SLS + fine particulate solution and application of SLS + fine particulate solution + test product, the TEWL was measured by means of a vapometer (Delfin Technologies Ltd, Finland) before application, right after application, in one day after application, in two days after application and in seven days after application. At every measurement, the TEWL on the same region was measured three times, such that an average thereof was used as evaluation data. Results of the degree of skin erythema measured for 22 subjects were indicated as average ± standard error (SE), which were shown in a following Table 40.

**[Table 40]**

| Classification | Application of SLS + fine particulate solution | | Application of SLS + fine particulate solution + test product | | Significance probability between application of SLS + fine particulate solution and application of SLS + fine particulate solution + test product |
|---|---|---|---|---|---|
| | Transepidermal water loss (TEWL) | Significance Probability | Transepidermal water loss (TEWL) | Significance Probability | |
| Test of within-subject effect | - | 0.000# | - | 0.000# | |
| Before application | 7.682±2.286 | - | 7.650±1.840 | - | |
| Right after application | 44.818±23.304 | - | 46.055±26.923 | - | 1 |
| 1 day after application | 58.227±34.123 | 0.001# | 47.500±24.141 | 0.921 | 0.213 |
| 2 days after application | 28.086±9.246 | 0.000# | 23.770±8.255 | 0.000# | 0.017** |
| 7 days after application | 18.368±8.376 | 0.000# | 11.968±3.439 | 0.000# | 0.005** |

| | | | | | |
|---|---|---|---|---|---|
| *: p<0.05 Post-hoc test results of a non-parametric test, which was repeatedly measured by means of a parametric method ANOVA according to a test of normality #: p<0.025 Results of conducting a Friedman test as a non-parametric method and then a post-hoc test as a non-parametric test method according to a test of normality +: p<0.05 Independent sample t test result **: p<0.05 Mann-Whitney U test result | | | | | |

As shown in Table 40, a measured value for the application of SLS + fine particulate solution was significantly increased (p<0.025) in one day after the application, and was significantly decreased (p<0.025) in two days and seven days after the application. A measured value for the application of SLS + fine particulate solution + test product was significantly decreased (p<0.025) in two days and seven days after the application. It was identified that the application of SLS + fine particulate solution + test product significantly decreased the TEWL (p<0.05) compared to the application of SLS + fine particulate in two days and seven days after the application.

Thus, it was identified that the application of the additional test product significantly decreased the TEWL, and it was identified that the application of SLS + fine particulate solution decreased the degree about 33.2% in seven days after the application compared to the application of SLS + fine particulate solution.

### (4) Intermediate conclusion

As seen in the above Experimental Example 8-2, it was identified that the test product all decreased a degree of skin redness (a*), an amount of skin erythema (E.I) or an amount of transepidermal water loss (TEWL), which are skin inflammation factors by means of fine particulate in seven days later, based on which it was identified that the test product alleviated skin inflammation caused from fine particulate.

## Claims

1. A composition for alleviating skin inflammation caused from yellow dust or fine particulate, comprising one or more extracts selected from the group consisting of *Citrus sunki* peel extract, *Sceptridium ternatum* (or *Botrychium ternatum)* extract and *Korthalsellajaponica* extract as an effective ingredient.

2. The composition for alleviating skin inflammation according to claim 1, **characterized in that** the extract is the *Korthalsellajaponica* extract.

3. The composition for alleviating skin inflammation according to claim 2, further comprising one or more of the *Citrus sunki* peel extract or the *Sceptridium ternatum* (or *Botrychium ternatum*) extract.

4. The composition for alleviating skin inflammation according to claim 1, **characterized in that** the extract is a mixture that combines the *Citrus sunki* peel extract, the *Sceptridium ternatum* (or *Botrychium ternatum)* extract and the *Korthalsellajaponica* extract.

5. The composition for alleviating skin inflammation according to claim 4, **characterized in that**, in case of the mixture, a composition ratio of the *Citrus sunki* peel extract: the *Sceptridium ternatum* (or *Botrychium ternatum)* extract: the *Korthalsellajaponica* extract is 1:1:1 to 2:1:1 by weight.

6. The composition for alleviating skin inflammation according to claim 1, **characterized in that** the extract is i) the *Citrus sunki* peel extract comprising one or more compounds selected from the group consisting of hesperidine, tetramethyl-O-isoscutellarein, nobiletin and tangeretin; ii) the *Sceptridium ternatum* (or *Botrychium ternatum*) extract comprising one or more compounds selected from the group consisting of quercetin-3-O-β-glucosyl(1→2)-α-rhamnoside-7-O-α-rhamnoside, quercetin-3-O-β-glucosyl(1→2)-α-rhamnoside-7-O-β-glucoside, ternatumoside VI, ternatumoside XI and ternatumoside XII; or iii) the *Korthalsellajaponica* extract comprising one or more compounds selected from the group consisting of lucenin-2, vicenin-2 and stellarin-2.

7. The composition for alleviating skin inflammation according to any one of claims 1 to 4, **characterized in that** the yellow dust or fine particulate increases a production amount of one or more cytokines selected from the group consisting of G-CSF, GM-CSF, GRO, GRO-α, IL-1α, IL-2, IL-3, IL-5, IL-6, IL-8, IL-15, MCP-1, MCP-2 and TNF-α.

8. The composition for alleviating skin inflammation according to any one of claims 1 to 4, **characterized in that** the fine particulate comprises one or more fine particulates selected from the group consisting of TSP (total suspended particle), PM10 (particulate matter 10) and PM2.5 (particulate matter 2.5).

9. The composition for alleviating skin inflammation according to any one of claims 1 to 4, **characterized in that** the extract inhibits a production of NO.

10. The composition for alleviating skin inflammation according to any one of claims 1 to 4, **characterized in that** the extract reduces a production amount of one or more cytokines selected from the group consisting of IL-1α, IL-2, IL-6, IL-8, GRO-α, GM-CSF and TNF-α.

11. The composition for alleviating skin inflammation according to any one of claims 1 to 4, **characterized in that** the extract is comprised by 0.001 to 30 wt% compared to the total weight of the composition.

12. The composition for alleviating skin inflammation according to any one of claims 1 to 4, **characterized in that** the skin inflammation is contact dermatitis.

13. A method for alleviating skin inflammation, comprising a step of applying a composition of any one of claims 1 to 4 to skin.

14. A method for decreasing a degree of skin redness, an amount of skin erythema or an amount of transepidermal water loss, comprising a step of applying a composition of any one of claims 1 to 4 to skin.
